(19) Europäisches Patentamt
European Patent Office
Office européen des brevets

(11) **EP 4 503 042 A1**

(12) **EUROPEAN PATENT APPLICATION**
published in accordance with Art. 153(4) EPC

(43) Date of publication:
**05.02.2025 Bulletin 2025/06**

(21) Application number: **23778772.6**

(22) Date of filing: **23.01.2023**

(51) International Patent Classification (IPC):
**G16C 20/30** (2019.01)

(52) Cooperative Patent Classification (CPC):
**G16C 20/30**

(86) International application number:
**PCT/JP2023/001945**

(87) International publication number:
**WO 2023/188733 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.03.2022 JP 2022058003**

(71) Applicant: **Panasonic Intellectual Property
Management Co., Ltd.
Kadoma-shi, Osaka 571-0057 (JP)**

(72) Inventors:
• **YOKOYAMA, Tomoyasu
Kadoma-shi, Osaka 571-0057 (JP)**
• **ICHIKAWA, Kazuhide
Kadoma-shi, Osaka 571-0057 (JP)**

(74) Representative: **Grünecker Patent- und
Rechtsanwälte
PartG mbB
Leopoldstraße 4
80802 München (DE)**

(54) **INFORMATION PROCESSING METHOD, INFORMATION PROCESSING SYSTEM, AND PROGRAM**

(57)    An information processing method is an information processing method performed by a computer, and includes a step (S101) of acquiring first information concerning polyhedra; a step (S101) of acquiring second information concerning atoms arranged in the polyhedra; a step (S 102 to S 104) of generating third information indicative of a crystal structure that can be taken in a case where the atoms are arranged in a three-dimensional structure in which the polyhedra are arranged on the basis of the first information and the second information; and a step (S 105) of outputting the generated third information.

FIG. 11

EP 4 503 042 A1

**Description**

Technical Field

The present disclosure relates to a technique for generating a crystal structure, and others.

Background Art

It is important to predict a crystal structure in order to search for a high-function material concerning a composition for which no experimental report has been made. In particular, in a case where a physical property of a material in which a physical property of a crystal structure such as electron conduction, ion conduction, heat conduction, or synthesizability is dominant is targeted, prediction accuracy of the crystal structure influences a screening result.

Patent Literature 1 and Patent Literature 2 disclose a method for generating a molecular structure on the basis of a feature vector of a structure.

Non Patent Literature 1 discloses a method for generating a structure on the basis of symmetry.

Citation List

Patent Literature

PTL 1: U. S. Patent Application Publication No. 2019/0286791
PTL 2: Japanese Unexamined Patent Application Publication No. 2021-81769 Non Patent Literature

NPL 1: S. Fredericks, K. Parrish, D. Sayre et al. Computer Physics Communications 261 (2021) 107810

Summary of Invention

The present disclosure provides an information processing method that makes it easy to generate a crystal structure with high prediction accuracy, and others.

An information processing method according to an aspect of the present disclosure is an information processing method performed by a computer, and includes acquiring first information concerning polyhedra; acquiring second information concerning atoms arranged in the polyhedra; generating third information indicative of a crystal structure that can be taken in a case where the atoms are arranged in a three-dimensional structure in which the polyhedra are arranged on the basis of the first information and the second information; and outputting the generated third information.

According to the present disclosure, it is easy to generate a crystal structure with high prediction accuracy.

Brief Description of Drawings

[Fig. 1] Fig. 1 illustrates an example of a three-dimensional structure generated from polyhedral.
[Fig. 2] Fig. 2 illustrates an example of a crystal structure generated from a three-dimensional structure.
[Fig. 3] Fig. 3 illustrates another example of a crystal structure generated from a three-dimensional structure.
[Fig. 4] Fig. 4 illustrates an example of a crystal structure generated from a periodic graph.
[Fig. 5] Fig. 5 is a block diagram illustrating an overall configuration including an information processing system according to Embodiment 1.
[Fig. 6] Fig. 6 illustrates an example of three-dimensional structure data stored in a first storage unit.
[Fig. 7] Fig. 7 illustrates an example of third information stored in a second storage unit.
[Fig. 8] Fig. 8 illustrates an image displayed on a display unit in a first use example of Embodiment 1.
[Fig. 9] Fig. 9 illustrates an image displayed on the display unit in the first use example of Embodiment 1.
[Fig. 10] Fig. 10 illustrates an image displayed on the display unit in a second use example of Embodiment 1.
[Fig. 11] Fig. 11 is a flowchart illustrating an operation example of the information processing system according to Embodiment 1.
[Fig. 12] Fig. 12 illustrates an example of arrangement pattern candidates.
[Fig. 13] Fig. 13 illustrate an example of a crystal structure corresponding to an arrangement pattern candidate.
[Fig. 14] Fig. 14 illustrates another example of the arrangement pattern candidate.

[Fig. 15] Fig. 15 illustrates another example of a crystal structure corresponding to the arrangement pattern candidate.

[Fig. 16] Fig. 16 illustrates an example of an arrangement of atoms in an arrangement pattern candidate.

[Fig. 17] Fig. 17 is a sequence diagram illustrating an operation example of the information processing system and the display unit, the first storage unit, and the second storage unit according to Embodiment 1.

[Fig. 18] Fig. 18 illustrates an example of an image displayed on a display unit according to Embodiment 2.

[Fig. 19] Fig. 19 is a flowchart illustrating an operation example of an information processing system according to Embodiment 2.

[Fig. 20] Fig. 20 is a block diagram illustrating an overall configuration including an information processing system according to Embodiment 3.

[Fig. 21] Fig. 21 is a flowchart illustrating an operation example of the information processing system according to Embodiment 3.

[Fig. 22] Fig. 22 is a sequence diagram illustrating an operation example of the information processing system and a display unit, a first storage unit, and a second storage unit according to Embodiment 3.

[Fig. 23] Fig. 23 illustrates an example of a second image displayed on the display unit according to a first use example of Embodiment 3.

[Fig. 24] Fig. 24 illustrates an example of a third image displayed on the display unit according to the first use example of Embodiment 3.

[Fig. 25] Fig. 25 illustrate an image displayed on the display unit in a second use example of Embodiment 3.

[Fig. 26] Fig. 26 illustrates another image displayed on the display unit in the second use example of Embodiment 3.

[Fig. 27] Fig. 27 illustrates still another image displayed on the display unit in the second use example of Embodiment 3.

[Fig. 28] Fig. 28 illustrates an image displayed on the display unit in a case where another physical property is selected in the second use example of Embodiment 3.

[Fig. 29] Fig. 29 is a flowchart illustrating another operation example of the information processing system according to Embodiment 3.

[Fig. 30] Fig. 30 illustrates an image displayed on the display unit in a modification.

[Fig. 31] Fig. 31 illustrates an image displayed on the display unit in a modification.

[Fig. 32] Fig. 32 illustrates a specific example of a case where a periodic graph is converted into a three-dimensional structure.

Description of Embodiments

(Underlying Knowledge Forming Basis of the Present Disclosure)

A physical property of a material such as electron conduction, ion conduction, or heat conduction greatly depends on how an atom is coordinated with surrounding atoms, that is, an atom's local coordination environment. For example, in a case of an ionic crystal material, a cation is surrounded by a group of anions. A polyhedron formed by connecting centers of these anions is called a coordination polyhedron. For example, AgI takes a face-centered cubic lattice structure (a fcc-type structure) of low Ag ion conductivity and a body-centered cubic lattice structure (a bcc-type structure) of high Ag ion conductivity. In the fcc-type structure, I ions around an Ag ion are formed in such a manner that the structure is filled with a coordination polyhedron made up of a tetrahedron and an octahedron. Since the Ag ion is stable in an octahedral site and therefore cannot move to an adjacent tetrahedral site, the Ag ion is hard to conduct. On the other hand, in the bcc-type structure, the I ions around the Ag ion are formed in such a manner that the structure is filled with a coordination polyhedron made up of tetrahedra. Since all sites are equivalent, the Ag ion easily conducts.

As just described, a physical property that a material exhibits differs depending on a kind of filling coordination polyhedron. Therefore, if a crystal structure can be generated by designating a coordination polyhedron having a target physical property, an unknown material can be efficiently searched for. That is, a crystal structure that has not been reported before can be generated, and an unknown high-function material can be found.

However, conventionally, there is no method for generating a crystal structure upon input of a coordination polyhedron, and it is difficult to find a desired unknown material having target high functionality.

For example, Patent Literature 1 and Patent Literature 2 disclose a method for generating a new structure on the basis of a structure feature vector. However, Patent Literature 1 and Patent Literature 2 do not disclose a method for generating a new crystal structure on the basis of a coordination polyhedron.

Non Patent Literature 1 discloses a method for generating a new crystal structure on the basis of symmetry. However, Non Patent Literature 1 does not disclose a method for generating a new crystal structure on the basis of a coordination polyhedron.

In order to solve the above problem, an information processing method according to an aspect of the present disclosure is an information processing method performed by a computer, and includes acquiring first information concerning polyhedra; acquiring second information concerning atoms arranged in the polyhedra; generating third information indicative of a crystal structure that can be taken in a case where the atoms are arranged in a three-dimensional structure in which the polyhedra are arranged on the basis of the first information and the second information; and outputting the generated third information.

This makes it easy to generate a crystal structure with high prediction accuracy.

For example, the three-dimensional structure may be a structure in which the polyhedra are arranged without any gaps.

This makes it possible to generate a crystal structure having a space-filled structure in a three-dimensional space.

In the acquiring the first information, information concerning the three-dimensional structure in which the polyhedra are arranged without any gaps may be acquired as the first information.

This makes it possible to generate a crystal structure having a space-filled structure in a three-dimensional space.

The information concerning the three-dimensional structure may include at least one of information indicative of the three-dimensional structure, information indicative of a numerical sequence including a numeral or a character representing the three-dimensional structure, or information indicative of a periodic graph representing the three-dimensional structure.

This makes it possible to generate a crystal structure having a space-filled structure in a three-dimensional space by various methods.

In the generating the third information, the third information indicative of the crystal structure may be generated by using an arrangement pattern of the atoms that can be taken in the crystal structure.

This makes it possible to exhaustively generate a crystal structure.

The arrangement pattern may indicate that the atoms are arranged at vertexes of the polyhedra and/or insides of the polyhedra.

This makes it possible to exhaustively generate a crystal structure.

In the acquiring the second information, material information concerning a material containing the atoms arranged in the polyhedra may be acquired as the second information, and in the generating the third information, the third information indicative of the crystal structure which the material can take may be generated on the basis of the material information.

This makes it possible to generate a crystal structure, for example, under restriction of a material designated by a user, thereby making it easy to generate a crystal structure desired by the user.

In the acquiring the second information, element information indicative of a kind of each of the atoms may be further acquired as the material information, and in the generating the third information, the third information indicative of the crystal structure in which the atoms are arranged so that each kind indicated by the element information includes one or more atoms may be generated.

This makes it possible to generate a crystal structure, for example, under restriction of kinds of atoms designated by a user, thereby making it easy to generate a crystal structure desired by the user.

In the acquiring the second information, composition information concerning a composition of the material may be further acquired as the material information, and in the generating the third information, the third information indicative of the crystal structure having the composition indicated by the composition information may be generated.

This makes it possible to generate a crystal structure, for example, under restriction of a composition designated by a user, thereby making it easy to generate a crystal structure desired by the user.

In the acquiring the second information, atom number information indicative of the number of atoms arranged in the polyhedra may be further acquired as the material information, and in the generating the third information, the third information indicative of the crystal structure in which as many atoms as the number indicated by the atom number information are arranged may be generated.

This makes it possible to generate a crystal structure, for example, under restriction of the number of atoms designated by a user, thereby making it easy to generate a crystal structure desired by the user.

In the acquiring the first information, crystal structure information indicative of a base crystal structure, which is a base of the crystal structure, may be acquired as the first information, and in the acquiring the third information, the third information indicative of the crystal structure that can be taken in a case where the atoms are arranged in the base crystal structure indicated by the crystal structure information may be generated.

This makes it possible to generate a crystal structure, for example, under restriction of a base crystal structure designated by a user, thereby making it easy to generate a crystal structure desired by the user.

In the generating the third information, positions of vertexes of the polyhedra and positions of insides of the polyhedra in which the atoms are arranged are determined on the basis of the base crystal structure indicated by the crystal structure information.

This makes it possible to generate a crystal structure, for example, under restriction of a base crystal structure designated by a user, thereby making it easy to generate a crystal structure desired by the user.

The information processing method may further include calculating and outputting fourth information concerning a physical property of the crystal structure indicated by the third information by using at least one of ab initio calculation or a prediction model trained by machine learning.

This allows a user to efficiently search for an unknown material by referring to a physical property of a generated crystal structure.

In the generating the third information, the third information may be output only for the crystal structure having a predetermined physical property on the basis of the calculated fourth information.

This makes it possible to generate a crystal structure, for example, under restriction of a physical property of a crystal structure designated by a user, thereby making it easy to generate a crystal structure desired by the user.

An information processing system according to an aspect of the present disclosure includes a display that displays a first image for receiving input of first information concerning polyhedra and second information concerning atoms arranged in the polyhedra; and a display controller that causes a second image to be displayed on the display, the second image showing third information indicative of a crystal structure that can be taken in a case where the atoms are arranged in a three-dimensional structure in which the polyhedra are arranged, the third information being generated on the basis of the input first information and second information.

This allows a user to check a generated crystal structure with high prediction accuracy.

The first image may include a first input image for receiving input of the first information and a second input image for receiving input of the second information, and the display controller may cause the second input image to be displayed on the display, and causes the first input image to be displayed on the display on the basis of the input second information.

This allows a user to check a generated crystal structure with high prediction accuracy.

A program according to an aspect of the present disclosure causes a computer to perform processing including: acquiring first information concerning polyhedra; acquiring second information concerning atoms arranged in the polyhedra; generating third information indicative of a crystal structure that can be taken in a case where the atoms are arranged in a three-dimensional structure in which the polyhedra are arranged on the basis of the first information and the second information; and outputting the generated third information.

This makes easy to generate a crystal structure with high prediction accuracy.

The present disclosure may be realized as a computer program that causes a computer to perform the characteristic processing included in the information processing method of the present disclosure. Needless to say, such a computer program can be distributed on a computer-readable non-transitory recording medium such as a CD-ROM or over a communication network such as the Internet.

That is, according to the technique of the present disclosure, by inputting information on polyhedra, it is possible to generate a crystal structure configured such that atoms are arranged in a three-dimensional structure made up of a combination of the input polyhedra. The three-dimensional structure is a structure in a three-dimensional space, especially, a space-filled structure in a three-dimensional space, in other words, a structure configured such that a three-dimensional space is filled with polyhedra without any gaps. Note that the expression "filled with polyhedra without any gaps" means that each of the polyhedra is arranged so that each vertex of a face thereof that is in contact with another polyhedron is at the same height as a corresponding vertex of a face of the other polyhedron that is in contact with the polyhedron.

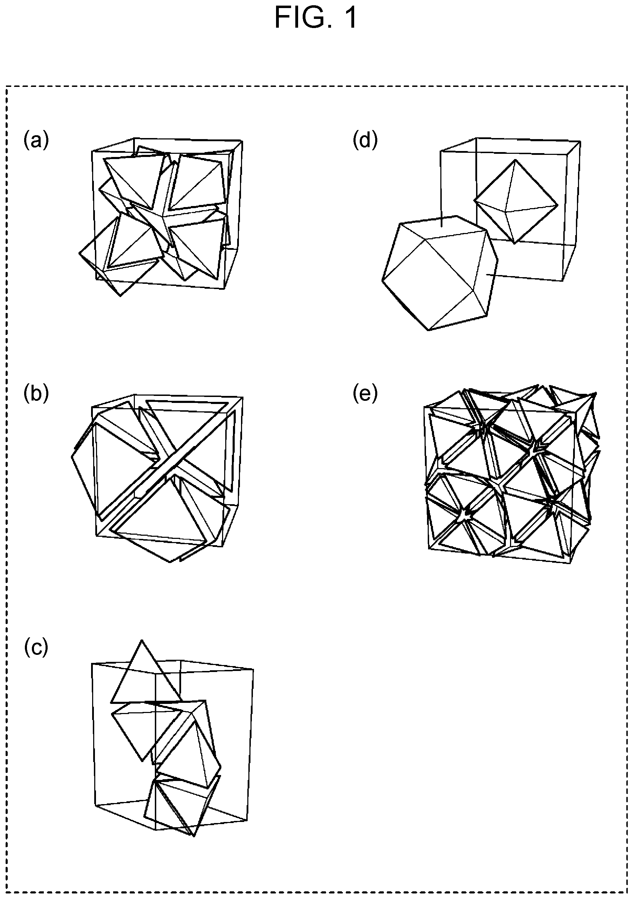

A wide variety of three-dimensional structures can be generated by combining polyhedra. Fig. 1 illustrates an example of a three-dimensional structure generated from polyhedra. Fig. 1(a) illustrates a face-centered cubic lattice structure (a fcc-type structure) made up of regular tetrahedra and regular octahedra. Fig. 1(b) illustrates a body-centered cubic lattice structure (a bcc-type structure) made up of regular tetrahedra. Fig. 1(c) illustrates a hexagonal close-packed structure (an hcp-type structure) made up of regular tetrahedra and regular octahedra. Fig. 1(d) illustrates a perovskite structure made up of one regular octahedron and cuboctahedra. Note that although one cuboctahedron is illustrated in Fig. 1(d), cuboctahedra are actually arranged around the regular octahedron at the center without any gaps. Fig. 1(e) illustrates an $MgCu_2$-type structure made up of regular tetrahedra.

Since a structure of an inorganic material can be regarded as a three-dimensional structure, the technique of the present disclosure that can generate a crystal structure upon input of information on polyhedra, for example, a three-dimensional structure is very effective for searching for an unknown material.

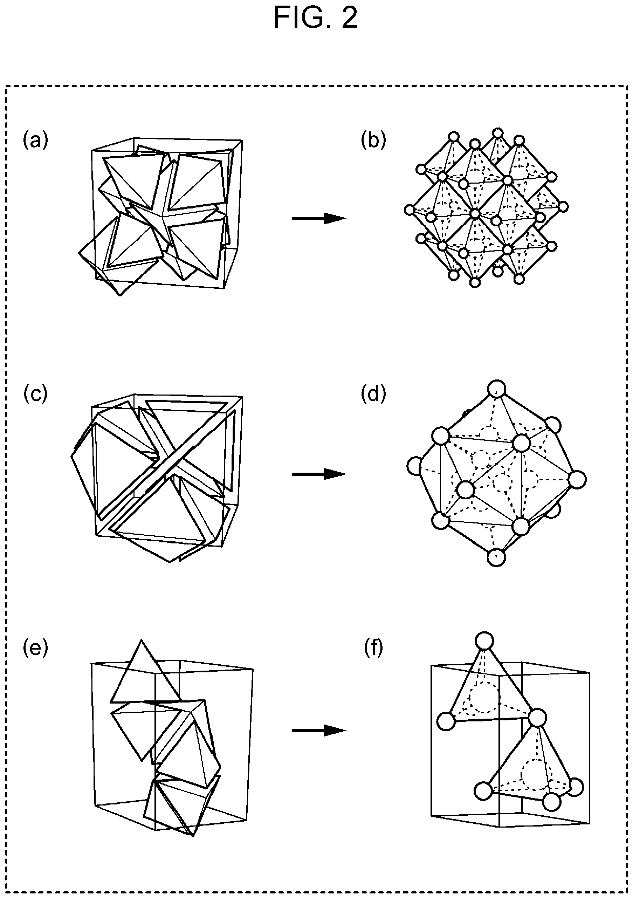

Fig. 2 illustrates an example of a crystal structure generated from a three-dimensional structure. For example, in a case where Cl is disposed at vertex sites, Na is disposed at center sites of octahedra, and vacancy is disposed at center sites of tetrahedra in a three-dimensional structure having a fcc-type structure illustrated in Fig. 2(a), NaCl having a rock-salt-type structure illustrated in Fig. 2(b) is obtained. For example, in a case where I is disposed at vertex sites and Ag is disposed at center sites of tetrahedra in a three-dimensional structure having a bcc-type structure illustrated in Fig. 2(c), AgI having an AgI-type structure illustrated in Fig. 2(d) is obtained. For example, in a case where O is disposed at vertex sites, Zn is disposed at center sites of one or some tetrahedra, and vacancy is disposed at center sites of remaining tetrahedra and center sites of octahedra in a three-dimensional structure having a hcp-type structure illustrated in Fig. 2(e), ZnO having a wurtzite-type structure illustrated in Fig. 2(f) is obtained.

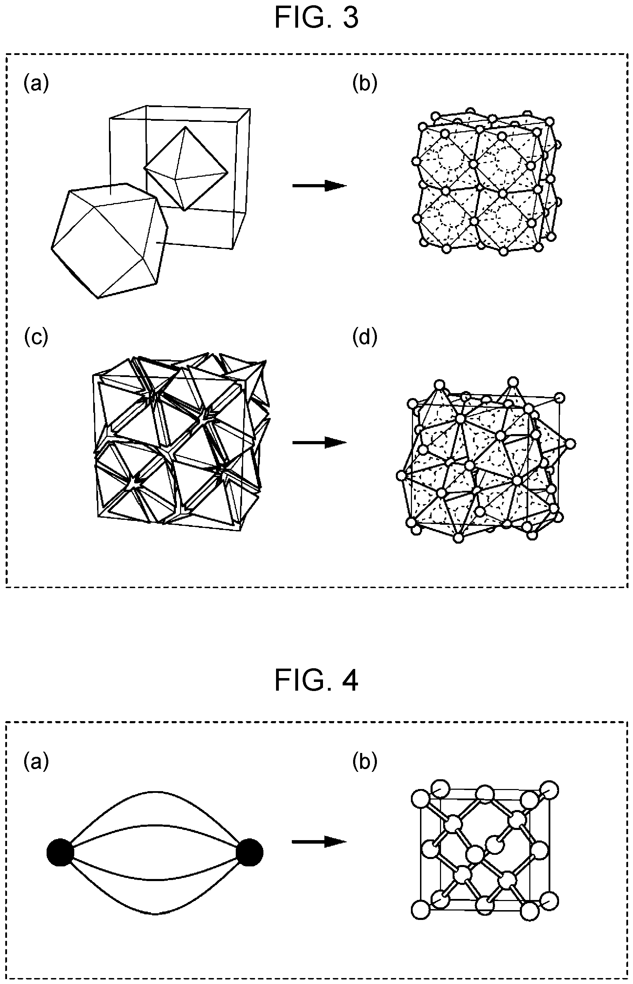

Fig. 3 illustrates another example of a crystal structure generated from a three-dimensional structure. For example, in a case where O is disposed at vertex sites, Ti is disposed at a center site of an octahedron, and Sr is disposed at center sites of truncated octahedra in a three-dimensional structure having a perovskite-type structure illustrated in Fig. 3(a), $SrTiO_3$ having a perovskite-type structure illustrated in Fig. 3(b) is obtained. For example, in a case where S and Cl are disposed at vertex sites and Li and P are disposed at center sites of tetrahedra in a three-dimensional structure having an $MgCu_2$-type structure illustrated in Fig. 3(c), $Li_6PS_5Cl$ having an argyrodite-type structure illustrated in Fig. 3(d) is obtained.

As described above, according to the technique of the present disclosure, a crystal structure based on a three-dimensional structure, that is, a coordination polyhedron can be generated by disposing an element or vacancy at a vertex site and a center site of each polyhedron in a three-dimensional structure based on input information on polyhedra. Therefore, according to the technique of the present disclosure, a crystal structure adapted to reality can be generated, and it is easy to generate a crystal structure with high prediction accuracy.

In general, a molecular structure can be expressed as a graph. That is, a molecular structure can be expressed as a graph structure expressing "atoms" constituting a compound as "nodes" and expressing "a bond between atoms" as an "edge" connecting the nodes. For example, an example in which a molecular structure is generated by expressing the molecular structure as a graph is disclosed in Patent Literature 2 (Japanese Unexamined Patent Application Publication No. 2021-081769).

On the other hand, a crystal structure needs to be expressed not by a normal graph, but by a periodic graph. The periodic graph is also called a crystal net and is a three-dimensionally periodic graph. The expression "three-dimensionally periodic" means that three linear independent translations exist. In general, by defining an atomic bond in a crystal structure, the crystal structure can be converted into a periodic graph. Furthermore, a periodic graph can be uniquely converted into a crystal structure by using the Kotani-Sunada theory (Kotani-Sunada, 2000, Trans. Amer. Mat). For example, as illustrated in Fig. 4(a), a periodic graph having two independent nodes and four edges connecting these nodes are interchangeable with a diamond-like structure illustrated in Fig. 4(b). In other words, the periodic graph is a graph that can be converted into a crystal structure that can be taken in a case where atoms are arranged in a three-dimensional structure in which polyhedra are arranged.

Embodiments are specifically described below with reference to the drawings.

Each of the embodiments described below illustrates a general or specific example of the present disclosure. Numerical values, shapes, materials, constituent elements, the way in which the constituent elements are disposed and connected, steps, the order of steps, and the like illustrated in the embodiments below are examples and do not limit the present disclosure. Among constituent elements in the embodiments below, constituent elements that are not described in independent claims indicating highest concepts are described as optional constituent elements. Furthermore, contents in the embodiments may be combined in all the embodiments. Each drawing is a schematic view and is not necessarily strict illustration. In the drawings, identical constituent elements are given identical reference signs.

Note that expression using a subscript is omitted in the composition formula in the drawings appearing in the embodiments below.

An information processing system according to an embodiment of the present disclosure may be configured such that all constituent elements are included in a single computer or may be configured as a system in which constituent elements are distributed into computers.

(Embodiment 1)

An information processing system 100 (an information processing method, or a program) according to Embodiment 1 of the present disclosure is described in detail below with reference to the drawings.

[Information Processing System]

First, a configuration of the information processing system used in Embodiment 1 is described.

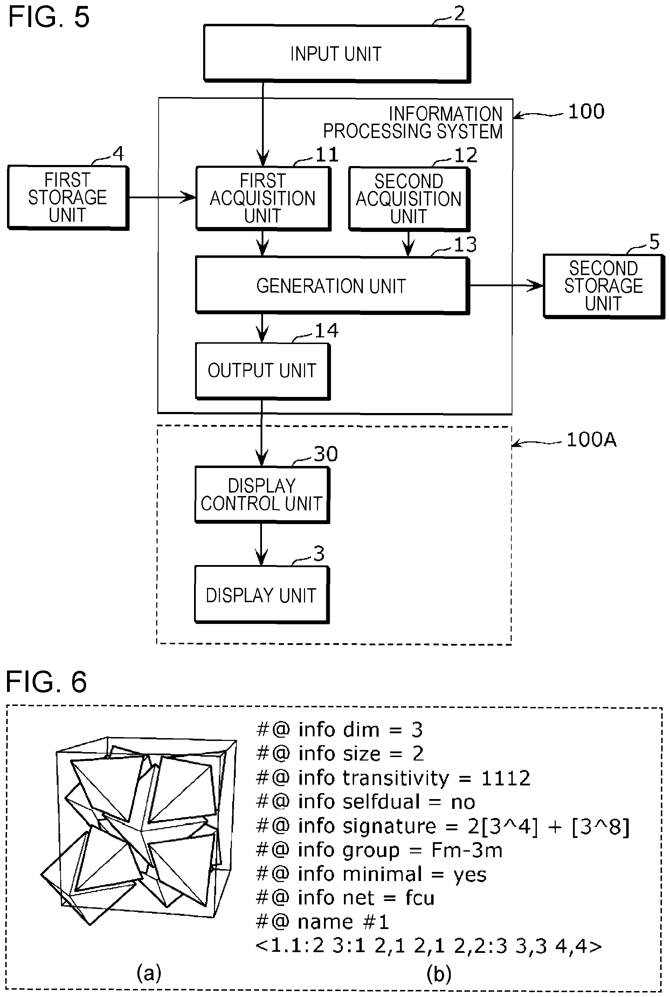

Fig. 5 is a block diagram illustrating an overall configuration including the information processing system 100 according to Embodiment 1. The information processing system 100 is, for example, a computer such as a personal computer or a server. That is, the information processing system 100 may be, for example, realized by cloud computing. In Embodiment 1, it is assumed that the information processing system 100 is a desktop computer.

The information processing system 100 includes a first acquisition unit 11, a second acquisition unit 12, a generation unit 13, and an output unit 14. Furthermore, to the information processing system 100, an input unit 2, a display control unit 30, a display unit 3, a first storage unit 4, and a second storage unit 5 are connected. The input unit 2, the display control unit 30, and the display unit 3 are, for example, realized by an information terminal used by a user such as a smartphone, a tablet terminal, or a personal computer. The input unit 2, the display control unit 30, and the display unit 3 may be an input unit, a display control unit, and a display unit included in an information terminal used by a user.

The input unit 2, the display control unit 30, the first storage unit 4, and the second storage unit 5 may be connected to the information processing system 100 over a Local Area Network (LAN) or the like or may be connected to the information processing system 100 over a network such as the Internet.

The input unit 2 is an input interface that receives user's input and is, for example, a keyboard, a touch sensor, a touch pad, a mouse, or the like. The input unit 2 receives user's input operation and outputs a signal according to the input operation to the information processing system 100. Although the display unit 3 and the input unit 2 are independent of each other in the present disclosure, the display unit 3 and the input unit 2 may be integral with each other (e.g., a touch panel). Furthermore, although the information processing system 100 includes neither the display unit 3 nor the input unit 2 in the present disclosure, the information processing system 100 may include the display unit 3 and the input unit 2.

The input unit 2 receives input of first information concerning polyhedra and input of second information concerning atoms arranged in the polyhedra. The first information is, for example, a three-dimensional structure. The first information may be, for example, kinds of polyhedra, the number of polyhedra, a permitted degree of distortion, permitted symmetry, or the like instead of a three-dimensional structure. In Embodiment 1, the first information is a three-dimensional structure. The second information can include, for example, kinds of elements disposed at center sites and vertex sites of the polyhedra, a composition ratio of the elements disposed at the center sites and the vertex sites of the polyhedra, symmetry, or the like. That is, the expression "atoms" encompasses a case where there are elements of one kind and a case where there are elements of two or more kinds. Note that the composition ratio of elements and symmetry are not essential information.

The display control unit 30 causes an image or the like to be displayed on the display unit 3 on the basis of information output from the output unit 14 of the information processing system 100.

The display unit 3 displays an image or the like under control of the display control unit 30. The display unit 3 is, for example, a liquid crystal display, a plasma display, an organic Electro-Luminescence (EL) display, or the like, but is not limited to this.

The first storage unit 4 is a recording medium in which a three-dimensional structure database is stored. The recording medium is, for example, a hard disk drive, a Random Access Memory (RAM), a Read Only Memory (ROM), a semiconductor memory, or the like. Note that such a recording medium may be volatile or may be non-volatile. The three-dimensional structure database includes data concerning a three-dimensional structure. Examples of the three-dimensional structure stored in the three-dimensional structure database include a fcc-type structure, a bcc-type structure, an hcp-type structure, a perovskite-type structure, and an $MgCu_2$-type structure. The three-dimensional structure data is used when the user input the first information by the input unit 2.

Fig. 6 illustrates an example of the three-dimensional structure data stored in the first storage unit 4. Fig. 6(a) illustrates a three-dimensional structure (in this example, a fcc-type structure), and Fig. 6(b) illustrates data describing the three-dimensional structure illustrated in Fig. 6(a) in a predetermined description format (in this example, a D-Symbol format). In the first storage unit 4, for example, an image showing a three-dimensional structure such as the one illustrated in Fig. 6(a) and data described in a predetermined description format such as the one illustrated in Fig. 6(b) are stored as three-dimensional structure data.

The second storage unit 5 is a recording medium in which third information indicative of a crystal structure generated by the generation unit 13 is stored. The recording medium is, for example, a hard disk drive, a Random Access Memory (RAM), a Read Only Memory (ROM), a semiconductor memory, or the like. Note that such a recording medium may be volatile or may be non-volatile.

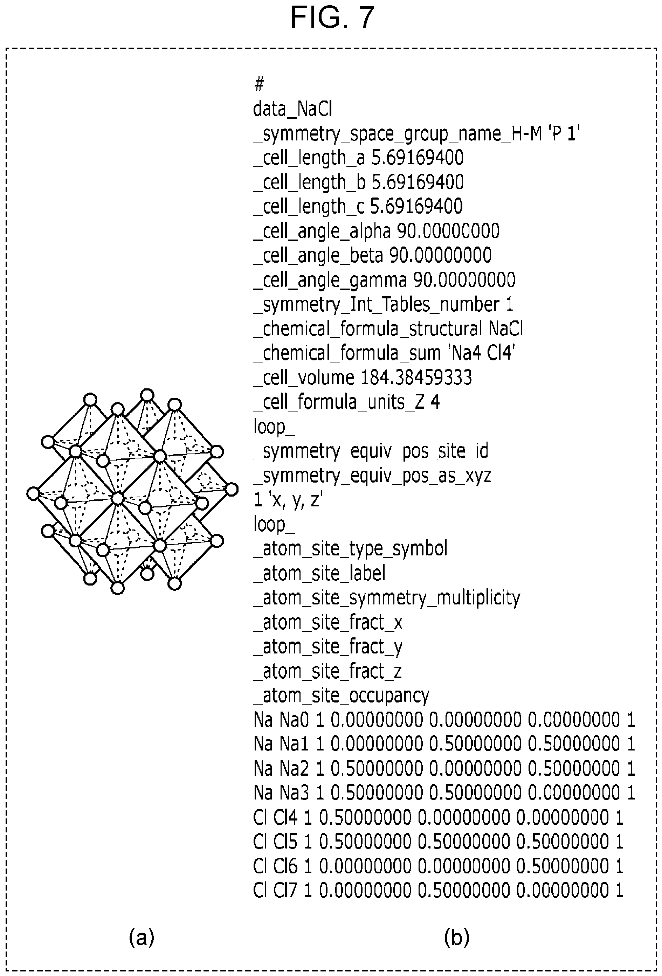

Fig. 7 illustrates an example of the third information stored in the second storage unit 5. Fig. 7(a) illustrates a crystal structure (a NaCl-type structure in this example) indicated by the third information, and Fig. 7(b) illustrates data in which the crystal structure illustrated in Fig. 7(a) is described in a predetermined description format (a cif format in this example). In the second storage unit 5, for example, an image showing a crystal structure such as the one illustrated in Fig. 7(a) and data described in a predetermined description format such as the one illustrated in Fig. 7(b) are stored as the third information. The third information includes, for example, an image, three-dimensional data, graph data, a space group, a Wyckoff label, a cell size, a lattice constant, an angle, a composition, an element kind, atom coordinates, or the like.

A file format (extension) of data stored in the second storage unit 5 is, for example, *.cif, *.pdb, *.ins, *.xyz, *.cc1, *.stl, *.wrl, *.pme, *.stin, *.p1, *.vasp, *.xtl, or the like.

The first acquisition unit 11 acquires the first information concerning polyhedra. The first acquisition unit 11 is a unit that executes a step of acquiring the first information in the information processing method of the present disclosure. Specifically, the first acquisition unit 11 acquires the first information input by the user by using the input unit 2. The user performs operation of inputting the first information while seeing a first image for receiving the first information and second information displayed on the display unit 3, as described later.

The second acquisition unit 12 acquires the second information concerning atoms arranged in polyhedra. The second acquisition unit 12 is a unit that executes a step of acquiring the second information in the information processing method of the present disclosure. Specifically, the second acquisition unit 12 acquires the second information input by the user by using the input unit 2. The user performs operation of inputting the second information while seeing the first image for receiving the first information and second information displayed on the display unit 3, as described later.

The generation unit 13 generates the third information indicative of a crystal structure that can be taken in a case where atoms are arranged in a three-dimensional structure where polyhedra are arranged on the basis of the first information acquired by the first acquisition unit 11 and the second information acquired by the second acquisition unit 12. The generation unit 13 is a unit that executes a step of generating the third information in the information processing method of the present disclosure. In Embodiment 1, the generation unit 13 performs processing for generating the third information indicative of a crystal structure by using an arrangement pattern of atoms that can be taken in a crystal structure. The arrangement pattern indicates that atoms are arranged at vertexes of polyhedra and/or insides (centers) of the polyhedra. In other words, the arrangement pattern of Embodiment 1 is obtained by a combination of positions of atoms that can be taken in a crystal structure. Details of the above processing will be described later.

The output unit 14 causes an image or the like to be displayed on the display unit 3 by outputting the image or the like to the display control unit 30. Furthermore, the output unit 14 outputs the third information generated by the generation unit 13. The output unit 14 is a unit that executes a step of outputting the third information in the information processing method of the present disclosure. Specifically, the output unit 14 outputs the second information by causing a second image indicative of the third information generated by the generation unit 13 to be displayed on the display unit 3. The user performs operation of selecting the third information to be stored in the second storage unit 5 while seeing the second image displayed on the display unit 3, as described later.

[Use Examples]

Examples of use of the information processing system 100 according to Embodiment 1 are listed below. In the description of a second use example below, description of points identical to a first use example is omitted.

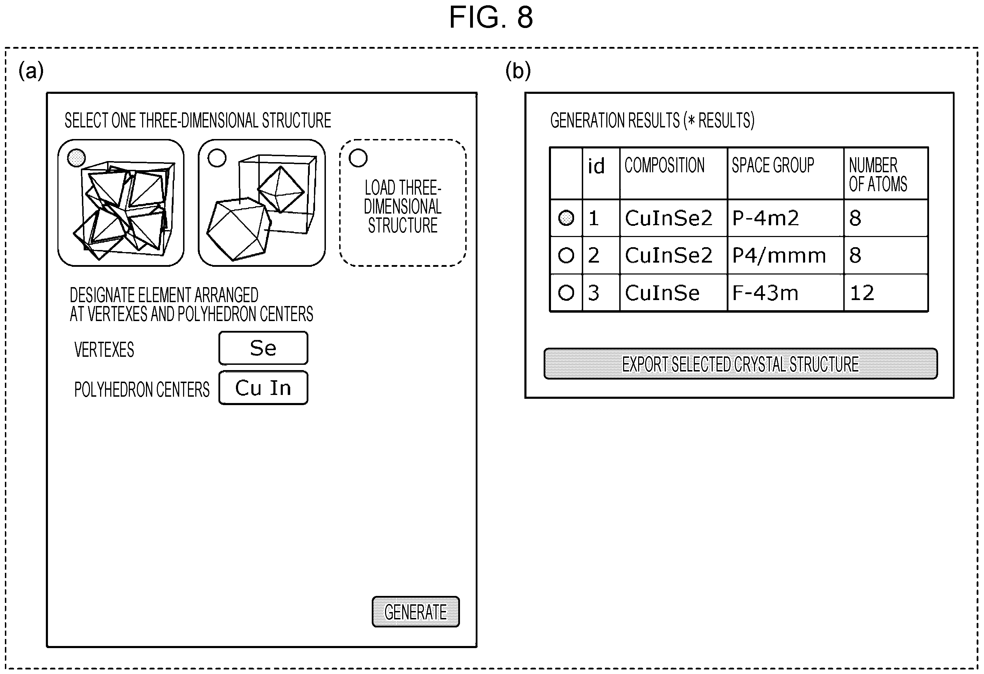

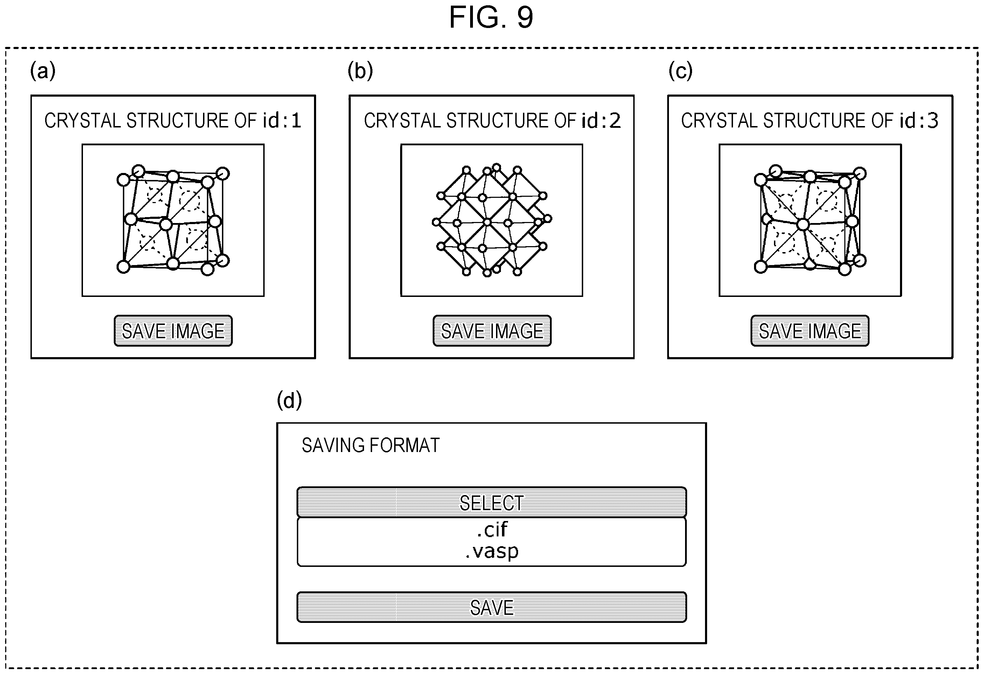

Figs. 8 and 9 each illustrate an image displayed on the display unit 3 in the first use example of Embodiment 1. Fig. 8(a) illustrates an example of the first image displayed on the display unit 3. The first image is displayed on the display unit 3 by the output unit 13 by reading out the three-dimensional structure data stored in the first storage unit 4. In the first use example, the first image includes a three-dimensional structure selection region for selecting a three-dimensional structure, an arrangement designation region for selecting an arrangement of atoms, and an execution icon "GENERATE".

In the three-dimensional structure selection region, three-dimensional structures that can be selected by a user and selection buttons corresponding to the three-dimensional structures are displayed. Note that names of the three-dimensional structures may be displayed in the three-dimensional structure selection region. In the three-dimensional structure selection region, each three-dimensional structure may be displayed not as a still image, but as a moving image. The user selects a three-dimensional structure in the three-dimensional structure selection region. As a result, the first acquisition unit 11 (in the step of acquiring the first information) acquires, as the first information, information concerning a three-dimensional structure in which polyhedra are arranged without any gaps. In this case, when the user selects the execution icon, the generation unit 13 (in the step of generating the third information) generates third information indicative of a crystal structure based on the selected three-dimensional structure. In the example illustrated in Fig. 8(a), the user selects a fcc-type structure. Accordingly, in this case, the generation unit 13 generates third information indicative of a crystal structure based on the fcc-type structure.

Note that, for example, in a case where the user possesses data concerning a three-dimensional structure that is not included in options in the three-dimensional structure selection region, the user may input this three-dimensional structure. In the example illustrated in Fig. 8(a), when the user selects a field captioned "LOAD THREE-DIMENSIONAL STRUCTURE" in the three-dimensional structure selection region, the three-dimensional structure possessed by the user can be acquired by the first acquisition unit 11.

In the arrangement designation region, a textbox for designating an element (atom) disposed at vertexes of each polyhedron and a textbox for designating an element (atom) disposed at a center of each polyhedron are displayed. The user inputs a desired element in each textbox. Note that in a case where the user wants to arrange vacancy at a center of each polyhedron, the user need just leave a corresponding textbox blank. As a result, the second acquisition unit 12 (in the step of acquiring the second information) acquires, as the second information, material information concerning a material having atoms arranged in polyhedra, more specifically, element information indicative of kinds of atoms arranged in the polyhedra. In this case, when the user selects the execution icon, the generation unit 13 (in the step of generating the third information) generates third information indicative of a crystal structure which the material can take, more specifically, a crystal structure in which atoms of kinds indicated by the element information are arranged on the basis of the material information. In the example illustrated in Fig. 8(a), the user inputs "Se" as an element arranged at vertexes of polyhedra and "Cu" and "In" as elements arranged at centers of the polyhedra. Note that the user may select an element such as "Se", "Cu", or "In" from a periodic table or from among preset candidate elements. Accordingly, in this case, the generation unit 13 generates third information indicative of a crystal structure in which "Se" is arranged at the vertexes of the polyhedra and "Cu" or "In" is arranged at the centers of the polyhedra.

Fig. 8(b) illustrates an example of the second image displayed on the display unit 3. The second image is displayed on the display unit 3 after the user selects the execution icon in the first image and the generation unit 13 generates third information concerning a crystal structure. The second image includes a table showing a list of crystal structures generated by the generation unit 13 and an execution icon "EXPORT SELECTED CRYSTAL STRUCTURE". The table shows, from the left, a column for selecting a crystal structure to be exported, a column showing an identification number (ID) of each crystal structure, a column showing a composition of the crystal structure, a column showing symmetry (a space group in this example) of the crystal structure, and a column showing the number of atoms included in a unit lattice (unit cell) of the crystal structure.

The user selects a crystal structure to be saved and selects the execution icon. As illustrated in Fig. 9(a) to (c), an image including a region showing the selected crystal structure and an execution icon "SAVE IMAGE" is thus displayed on the display unit 3. Fig. 9(a) illustrates an image displayed in a case where a crystal structure given identification number "1" is selected, Fig. 9(b) illustrates an image displayed in a case where a crystal structure given identification number "2" is selected, and Fig. 9(c) illustrates an image displayed in a case where a crystal structure given identification number "3" is selected.

Then, the user checks the selected crystal structure and, if there is no problem, selects the execution icon. As a result, an image including a selection region for selecting a saving format of the crystal structure and an execution icon "SAVE" is displayed on the display unit 3, as illustrated in Fig. 9(d). Although the user can select any one of ".cif" and ".vasp" in the example illustrated in Fig. 9(d), other saving formats may be selectable. When the user selects a desired saving format and then selects the execution icon, third information concerning the crystal structure selected by the user is saved in the second storage unit 5.

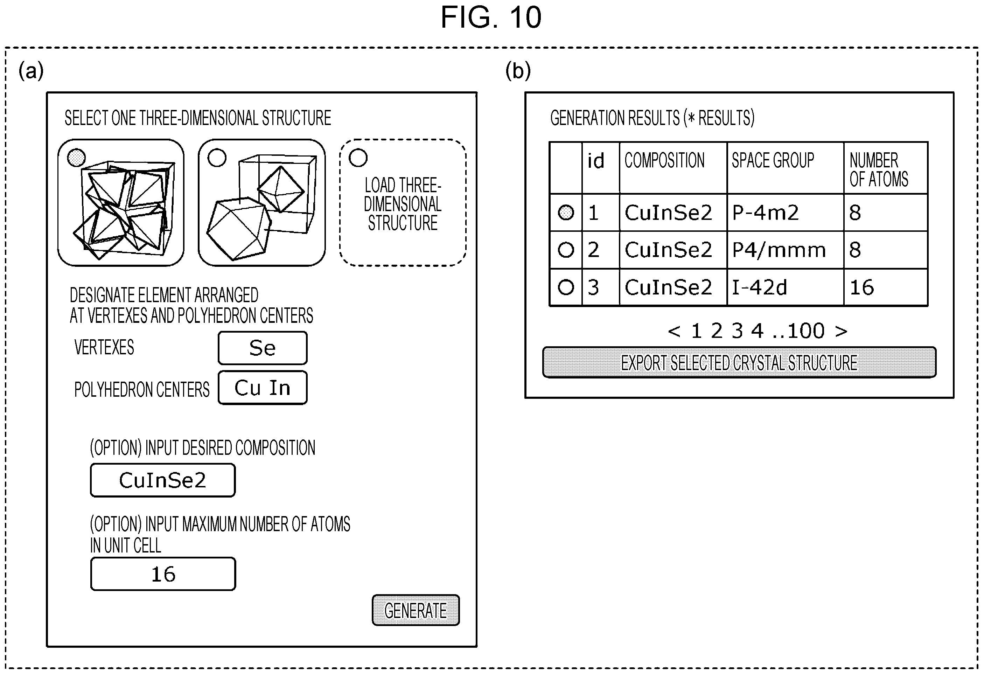

Fig. 10 illustrates an image displayed on the display unit 3 in the second use example of Embodiment 1. Fig. 10(a) illustrates an example of the first image displayed on the display unit 3. Fig. 10(b) illustrates an example of the second image displayed on the display unit 3. In the second use example, the first image further includes a composition designation region for designated a composition desired by the user and a maximum atom number designation region for designating a maximum value of the number of atoms in a unit lattice (unit cell) in a crystal structure, unlike the first use example.

In the composition designation region, a textbox for designating a composition of a crystal structure is displayed. The user inputs a desired composition in the textbox. As a result, the second acquisition unit 12 (in the step of acquiring the second information) further acquires, as the material information, composition information concerning a composition of a material. In this case, when the user selects the execution icon, the generation unit 13 (in the step of generating the third information) generates third information indicative of a crystal structure having a composition indicated by the composition information. In the example illustrated in Fig. 10(a), the user designates "$CuInSe_2$" as a composition of a crystal structure. Accordingly, in this case, the generation unit 13 generates third information indicative of a crystal structure having the composition "$CuInSe_2$". Accordingly, in the second image, the crystal structure having the composition "$CuInSe_2$" is displayed, as illustrated in Fig. 10(b).

In the maximum atom number designation region, a textbox for designating a maximum value of the number of atoms in a unit lattice of a crystal structure is displayed. The user inputs a desired number in the textbox. As a result, the second acquisition unit 12 (in the step of acquiring the second information) further acquires, as the material information, atom number information indicative of the number of atoms arranged in each of polyhedra (in this example, a maximum value of the number of atoms in a unit lattice). In this case, when the user selects the execution icon, the generation unit 13 (in the step of generating the third information) generates third information indicative of a crystal structure in which as many atoms as the number indicated by the atom number information are arranged (in this example, atoms are arranged so that the number of atoms in the unit lattice does not exceed the designated maximum value of the number of atoms). In the example illustrated in Fig. 10(a), the user designates "16" as a maximum value of the number of atoms in the unit lattice. Accordingly, in this case, the generation unit 13 generates third information indicative of a crystal structure in which the number of atoms in the unit lattice is "16" or less. Accordingly, in the second image, a crystal structure in which the number of atoms in the unit lattice is "16" or less is displayed, as illustrated in Fig. 10(b).

Note that although the first image includes both the composition designation region and the maximum atom number designation region in the second use example, the first image may include any one of these regions.

[Operation]

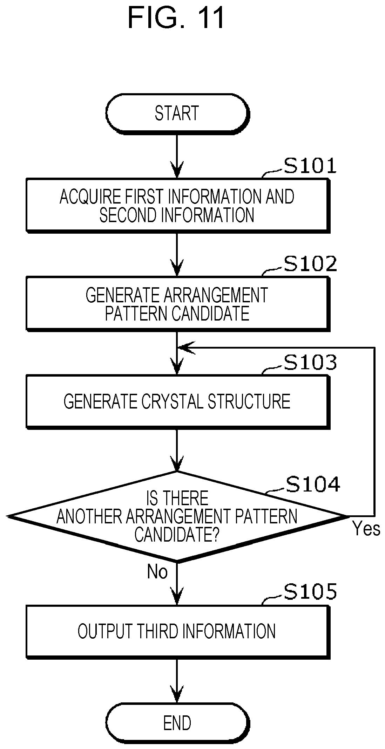

Operation of the information processing system 100 (i.e., the information processing method) according to Embodiment 1 is described below. Fig. 11 is a flowchart illustrating an operation example of the information processing system 100 according to Embodiment 1.

(Step S101)

The first acquisition unit 11 acquires the first information. As described above, the three-dimensional structure data stored in the first storage unit 4 is read out, and a user inputs (selects) the first information by using the input unit 2 while seeing the first image displayed on the display unit 3. In this way, the first information is acquired by the first acquisition unit 11. Note that the user may input original data by using the input unit 2 without referring to the first image, and thereby the first information may be acquired by the first acquisition unit 11.

The second acquisition unit 12 acquires the second information. As described above, the user inputs (selects) the second information by using the input unit 2 while seeing the first image displayed on the display unit 3. In this way, the second information is acquired by the second acquisition unit 12.

(Step S102)

The generation unit 13 performs processing for generating an arrangement pattern candidate on the basis of the acquired first information and second information. In this example, the generation unit 13 generates an arrangement pattern candidate for each of a pattern in which atoms are arranged at vertex sites of polyhedra and a pattern in which atoms are arranged at center sites of the polyhedra. When generating an arrangement pattern candidate, atoms may be randomly arranged or may be arranged efficiently according to a predetermined rule. One example of the predetermined rule is to arrange atoms so that symmetry of a space group is satisfied, as disclosed in P.V. Bushlanov, V.A. Blatov and A.R. Oganov "Topology-based crystal structure generator" Computer Physics Communications 236 (2019) 1-7.

(Step S103)

The generation unit 13 performs processing for generating a crystal structure for each arrangement pattern candidate. Specifically, the generation unit 13 generates a crystal structure by arranging atoms at vertexes of the polyhedra and centers of the polyhedra in accordance with contents of the arrangement pattern candidate.

(Step S104)

The generation unit 13 determines whether or not there is another arrangement pattern candidate for which a crystal structure has not been generated. In a case where it is determined that there is another arrangement pattern candidate (step S104: Yes), the generation unit 13 returns to step S103. In a case where a crystal structure has been generated for all arrangement pattern candidates (step S104: No), the processing of the generation unit 13 is completed. Then, the information processing system 100 (the information processing method) performs step S105.

(Step S105)

The output unit 14 performs processing for outputting the third information generated by the generation unit 13. In this example, the output unit 14 outputs the third information by displaying the second image indicative of the second information generated by the generation unit 13 on the display unit 3.

The display unit 3 may include the display control unit 30. The display unit 3 including the display control unit 30 may be referred to as a display unit 3A. The output unit 14 may output the third information generated by the generation unit 13 to the display unit 3A. The display unit 3A may thus display the second image indicative of the third information. That is, the output unit 14 may cause the second image indicative of the third information to be displayed on the display unit 3A.

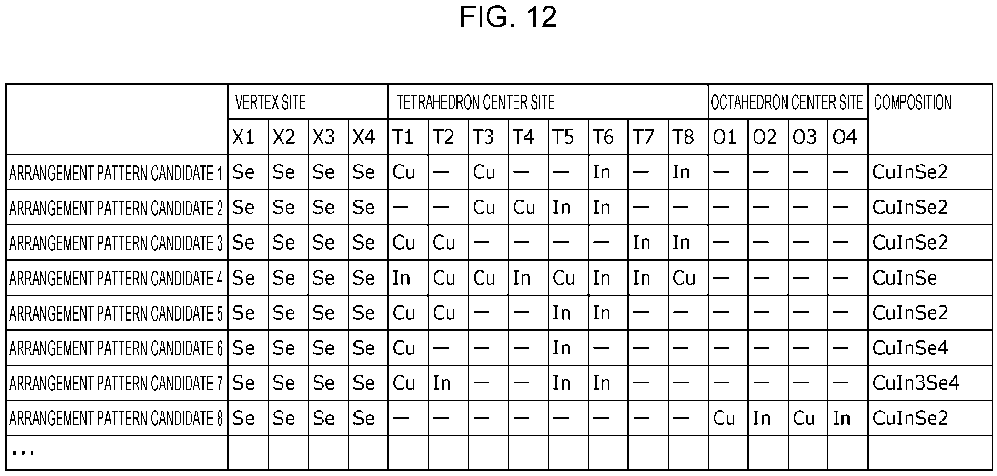

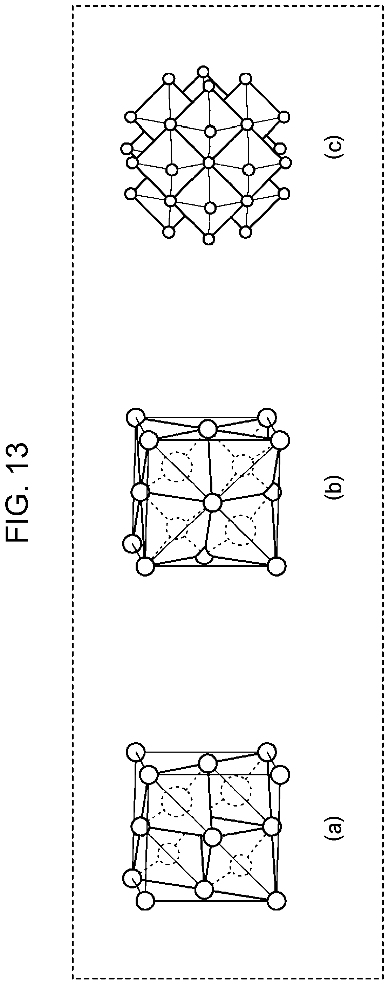

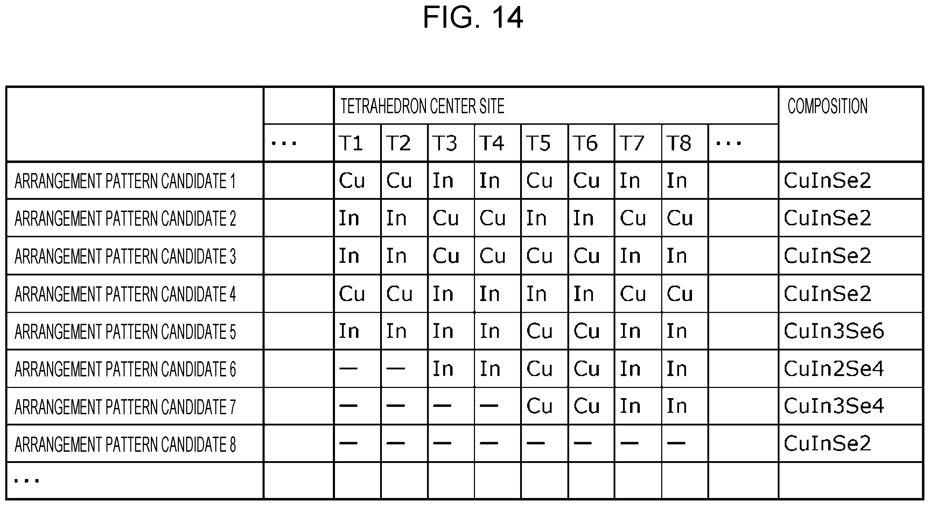

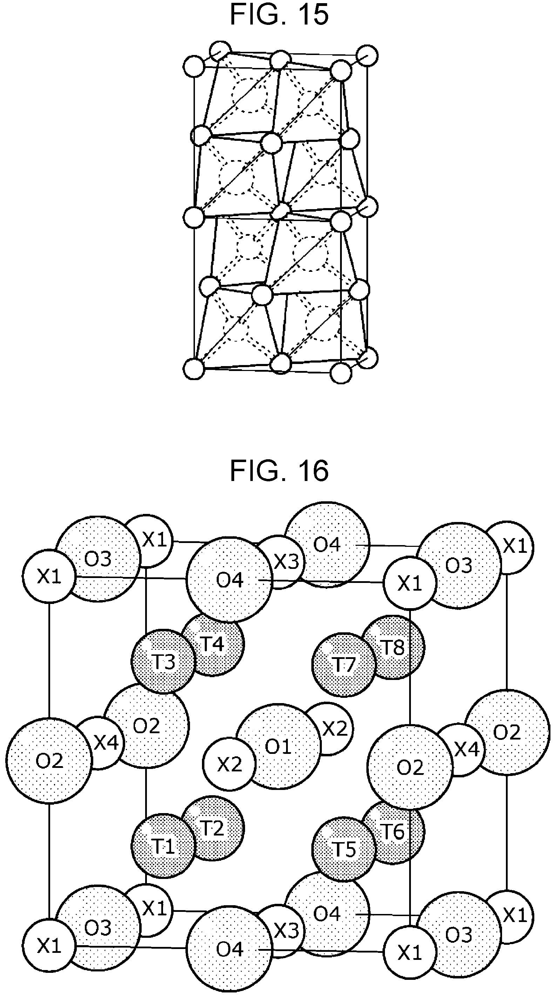

A specific example of the arrangement pattern candidate is described with reference to the drawings. Fig. 12 illustrates an example of arrangement pattern candidates. Fig. 13 illustrates an example of crystal structures corresponding to the arrangement pattern candidates. Fig. 14 illustrates another example of the arrangement pattern candidates. Fig. 15 illustrates another example of crystal structures corresponding to the arrangement pattern candidates. Fig. 16 illustrates an example of an arrangement of atoms in an arrangement pattern candidate.

Fig. 12 illustrates a table showing a list of arrangement pattern candidates of a fcc-type structure of a unit lattice (unit cell). The "vertex site" column in Fig. 12 shows an element (atom) arranged at vertexes of polyhedra. "A", "B", "C", and "D" in the "vertex site" column correspond to "X1", "X2", "X3", and "X4" in the arrangement of atoms illustrated in Fig. 16, respectively. The "tetrahedron center site" column in Fig. 12 shows an element (atom) or vacancy arranged at centers of tetrahedra. "A", "B", "C", "D", "E", "F", "G", and "H" in the "tetrahedron center site" column correspond to "T1", "T2", "T3", "T4", "T5", "T6", "T7", and "T8" in the arrangement of atoms illustrated in Fig. 16, respectively. The "octahedron center site" column in Fig. 12 shows an element (atom) or vacancy arranged at centers of octahedra. "A", "B", "C", and "D" in the "octahedron center site" column correspond to "O1", "O2", "O3", and "O4" in the arrangement of atoms illustrated in Fig. 16, respectively. The "composition" column in Fig. 12 shows a composition of a crystal structure.

For example, Fig. 13(a) illustrates a crystal structure corresponding to the "arrangement pattern candidate 1" in Fig. 12. That is, Fig. 13(a) illustrates a crystal structure whose composition is "CuInSe$_2$" and in which "Se" is arranged at vertexes of polyhedra, "Cu", "In", or vacancy is arranged at centers of tetrahedra, and vacancy is arranged at centers of octahedra. For example, Fig. 13(b) illustrates a crystal structure corresponding to the "arrangement pattern candidate 4" in Fig. 12. That is, Fig. 13(b) illustrates a crystal structure whose composition is "CuInSe" and in which "Se" is arranged at vertexes of polyhedra, "Cu" or "In" is arranged at centers of tetrahedra, and vacancy is arranged at centers of octahedra. For example, Fig. 13(c) illustrates a crystal structure corresponding to the "arrangement pattern candidate 8" in Fig. 12. That is, Fig. 13(c) illustrates a crystal structure whose composition is "CuInSe$_{-2}$" and in which "Se" is arranged at vertexes of polyhedra, vacancy is arranged at centers of tetrahedra, and "Cu" or "In" is arranged at centers of octahedra.

Fig. 14 illustrates a table showing a list of arrangement pattern candidates of a fcc-type structure of a $1 \times 1 \times 2$ supercell. In Fig. 14, the "vertex site" column and the "octahedron center site" column are omitted, and a part of the "tetrahedron center site" column is omitted. For example, Fig. 15 illustrates a crystal structure corresponding to the "arrangement pattern candidate 1" in Fig. 14. That is, Fig. 15 illustrates a crystal structure whose composition is "CuInSe$_2$" and in which "Se" is arranged at vertexes of polyhedra, "Cu", "In", or vacancy is arranged at centers of tetrahedra, and vacancy is arranged at centers of octahedra.

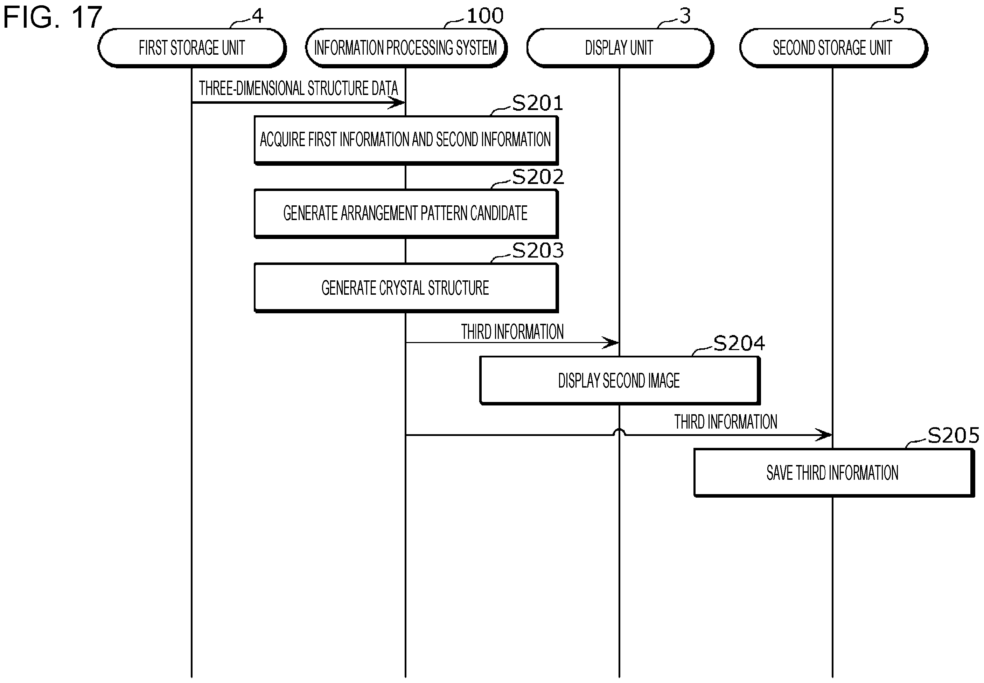

An operation example of the information processing system 100 and the display unit 3, the first storage unit 4, and the second storage unit 5 according to Embodiment 1 is described below with reference to the drawings. Fig. 17 is a sequence diagram illustrating an operation example of the information processing system 100 and the display unit 3, the first storage unit 4, and the second storage unit 5 according to Embodiment 1.

(Step S201)

The first acquisition unit 11 of the information processing system 100 acquires the first information. In this example, the three-dimensional structure data stored in the first storage unit 4 is read out, and a user inputs (selects) the first information by using the input unit 2 while seeing the first image displayed on the display unit 3. In this way, the first information is acquired by the first acquisition unit 11. Furthermore, the second acquisition unit 12 of the information processing system 100 acquires the second information. In this example, the user inputs (selects) the second information by using the input unit 2 while seeing the first image displayed on the display unit 3. In this way, the second information is acquired by the second acquisition unit 12.

(Step S202)

The generation unit 13 performs processing for generating an arrangement pattern candidate on the basis of the acquired first information and second information.

(Step S203)

The generation unit 13 performs processing for generating a crystal structure for each arrangement pattern candidate.

(Step S204)

The display unit 3 displays the second image indicative of the third information output from the output unit 14 of the information processing system 100. The display unit 3 may include the display control unit 30. The display unit 3 including the display control unit 30 may be referred to as a display unit 3A. The output unit 14 may display the second image indicative of the third information on the display unit 3A.

(Step S205)

When the user selects a crystal structure to be saved while seeing the second image displayed on the display unit 3, the information processing system 100 gives the third information indicative of the selected crystal structure to the second storage unit 5. In this way, the second storage unit 5 stores therein the third information indicative of the crystal structure selected by the user.

As described above, in Embodiment 1, a crystal structure based on a three-dimensional structure, that is, a coordination polyhedron can be generated by inputting information on polyhedra and arranging an element or vacancy at vertex sites and center sites of the polyhedra in the three-dimensional structure based on the input information on polyhedra. Therefore, in Embodiment 1, a crystal structure adapted to reality can be generated, and a crystal structure with high prediction accuracy is easily generated.

(Embodiment 2)

An information processing system (an information processing method, or a program) according to Embodiment 2 of the present disclosure is described in detail below with reference to the drawings. The information processing system according to Embodiment 2 is different from the information processing system 100 according to Embodiment 1 in that a first acquisition unit 11 acquires, as first information, crystal structure information indicative of a base crystal structure, which is a base of a crystal structure. The information processing system according to Embodiment 2 is different from the information processing system 100 according to Embodiment 1 in that a generation unit 13 generates third information indicative of a crystal structure that can be taken in a case where atoms are arranged in the base crystal structure indicated by the crystal structure information. Note that the information processing system according to Embodiment 2 includes the first acquisition unit 11, a second acquisition unit 12, the generation unit 13, and an output unit 14 and has a similar configuration to the information processing system 100 according to Embodiment 1, and therefore description of these constituent elements is omitted.

[Use Example]

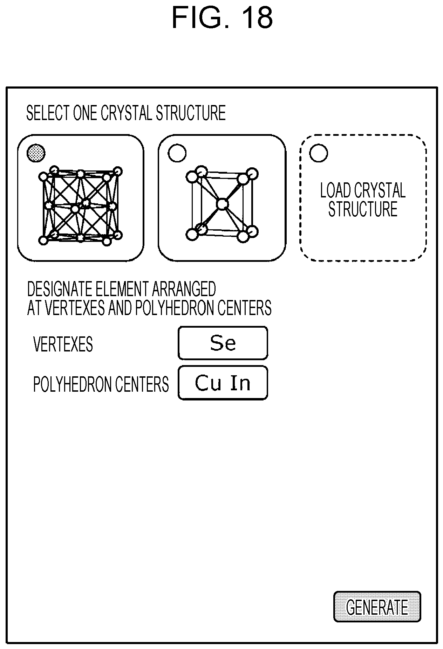

A use example of the information processing system according to Embodiment 2 is described below with reference to the drawings. Fig. 18 illustrates an example of an image displayed on a display unit 3 according to Embodiment 2. Fig. 18 illustrates an example of a first image displayed on the display unit 3. In Embodiment 2, the first image includes a crystal structure selection region for selecting a base crystal structure, which is a base of a crystal structure generated by the generation unit 13, instead of a three-dimensional structure selection region, unlike Embodiment 1.

In the crystal structure selection region, base crystal structures that can be selected by a user and selection buttons corresponding to the base crystal structures are displayed. Note that names of the base crystal structures may be displayed in the crystal structure selection region. In the crystal structure selection region, each base crystal structure may be displayed not as a still image, but as a moving image. A user selects a base crystal structure in the crystal structure selection region. As a result, the first acquisition unit 11 (in a step of acquiring first information) acquires, as the first information, crystal structure information indicative of a base crystal structure, which is a base of a crystal structure. In this case, when the user selects an execution icon, the generation unit 13 (in a step of generating third information) generates third information indicative of a crystal structure based on the selected base crystal structure.

Note that, for example, in a case where the user possesses data concerning a base crystal structure that is not included in options in the crystal structure selection region, the user may input this base crystal structure. In the example illustrated in Fig. 18, when the user selects a field "LOAD CRYSTAL STRUCTURE" in the crystal structure selection region, the base crystal structure possessed by the user can be acquired by the first acquisition unit 11.

[Operation]

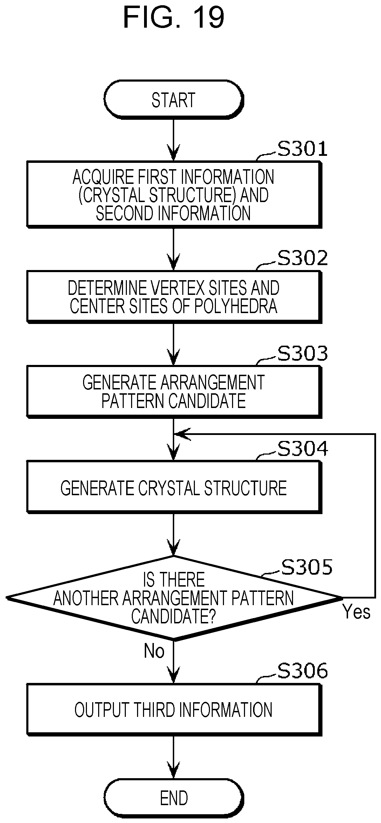

Operation of the information processing system (i.e., information processing method) according to Embodiment 2 is described below. Fig. 19 is a flowchart illustrating an operation example of the information processing system according to Embodiment 2.

(Step S301)

The first acquisition unit 11 acquires the first information. In Embodiment 2, the first information is the crystal structure information as described above, and a user inputs (selects) the first information by using the input unit 2 while seeing the first image displayed on the display unit 3. In this way, the first information is acquired by the first acquisition unit 11. The second acquisition unit 12 acquires the second information. The second information is similar to that in Embodiment 1.

(Step S302)

The generation unit 13 determines vertex sites of polyhedra and center sites of the polyhedra in the base crystal structure on the basis of the acquired first information. In other words, the generation unit 13 (in the step of generating the third information) determines positions of vertexes of the polyhedra and positions of insides (centers) of the polyhedra in which atoms are arranged on the basis of the base crystal structure indicated by the crystal structure information.

(Step S303)

The generation unit 13 performs processing for generating an arrangement pattern candidate on the basis of the acquired first information and second information, as in Embodiment 1.

(Step S304)

The generation unit 13 performs processing for generating a crystal structure for each arrangement pattern candidate, as in Embodiment 1.

(Step S305)

The generation unit 13 determines whether or not there is another arrangement pattern candidate for which a crystal structure has not been generated, as in Embodiment 1. In a case where there is another arrangement pattern candidate (step S305: Yes), the generation unit 13 returns to step S304. In a case where a crystal structure has been generated for all arrangement pattern candidates (step S305: No), the processing of the generation unit 13 is completed. Then, the information processing system (the information processing method) performs step S306.

(Step S306)

The output unit 14 performs processing for outputting the third information generated by the generation unit 13, as in Embodiment 1.

As described above, in Embodiment 2, a crystal structure can be generated by inputting information on a base crystal structure, which is a base of a crystal structure to be generated. Therefore, in Embodiment 2, a crystal structure adapted to reality can be generated, and a crystal structure with high prediction accuracy is easily generated, as in Embodiment 1.

(Embodiment 3)

An information processing system 200 (an information processing method, or a program) according to Embodiment 3 of the present disclosure is described in detail below with reference to the drawings. The information processing system 200 according to Embodiment 3 is different from the information processing system 100 according to Embodiment 1 in that fourth information concerning a physical property of a crystal structure generated by a generation unit 13 is calculated and output.

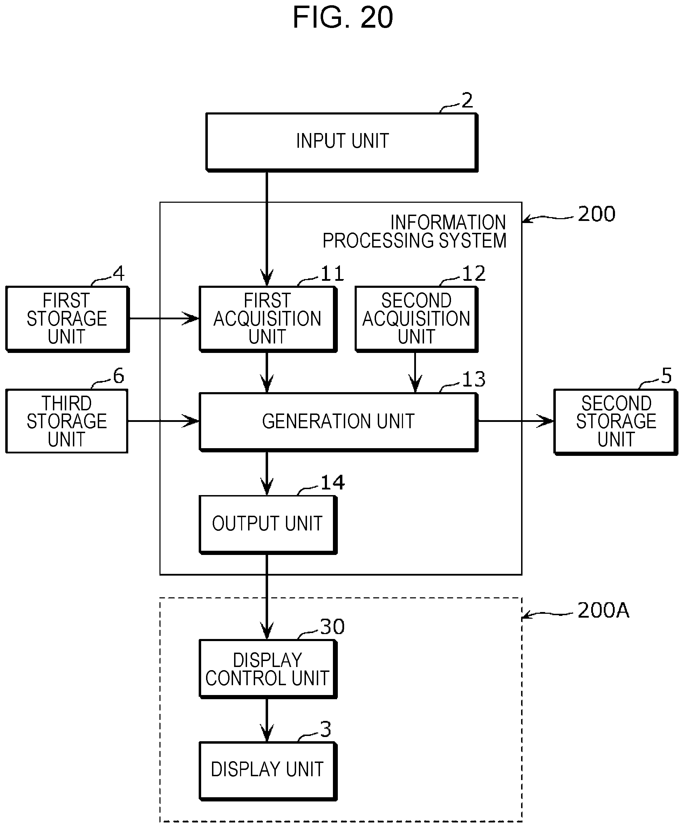

Fig. 20 is a block diagram illustrating an overall configuration including the information processing system 200 according to Embodiment 3. As illustrated in Fig. 20, a third storage unit 6 is further connected to the information processing system 200 according to Embodiment 3. In the information processing system 200 according to Embodiment 3, the generation unit 13 has a function of calculating fourth information concerning a physical property of a generated crystal structure. Note that description of a configuration identical to the information processing system 100 according to Embodiment 1 is omitted.

The third storage unit 6 is a recording medium in which a compound database concerning a physical property value (including a predicted value) of an existing material is stored. The recording medium is, for example, a hard disk drive, a Random Access Memory (RAM), a Read Only Memory (ROM), a semiconductor memory, or the like. Note that such a recording medium may be volatile or may be non-volatile.

The generation unit 13 predicts a physical property of a crystal structure on the basis of the generated crystal structure and the compound data read out from the third storage unit 6. Examples of the physical property can include a synthesizability index such as convex hull energy, an optical property such as a bandgap, an electronic property such as carrier mobility, a dielectric property such as relative permittivity, a transport property such as ion conductivity, and an electrochemical property such as a potential window. Examples of the physical property further include information concerning synthesis of a crystal structure. The information concerning synthesis can include a candidate for a raw material used for synthesis of a crystal structure, a condition of synthesis, or the like.

The physical property of the crystal structure can be, for example, calculated by ab initio calculation or by using a prediction model trained by machine learning. For example, the convex hull energy or bandgap of the crystal structure can be calculated by ab initio calculation. For example, the thermodynamic convex hull energy or bandgap of the crystal structure can be calculated (estimated) by using a prediction model.

The prediction model is a graph neural network using a graph structure as input. The graph neural network is, for example, a Crystal Graph Convolutional Neural Network (CGCNN), Material Graph Network (MEGNet), or the like. In this example, the prediction model is MEGNet. The MEGNet is a graph neural network that uses, as feature amounts, not only nodes (node points, vertexes) and edges (branches, sides), but also a global state amount representing a feature of a whole target system.

The prediction model is trained by machine learning by using a large number of learning datasets so as to output, upon input of any crystal structure, total energy of the crystal structure. The learning datasets include a crystal structure as input data and total energy corresponding to the crystal structure as correct answer data.

[Operation]

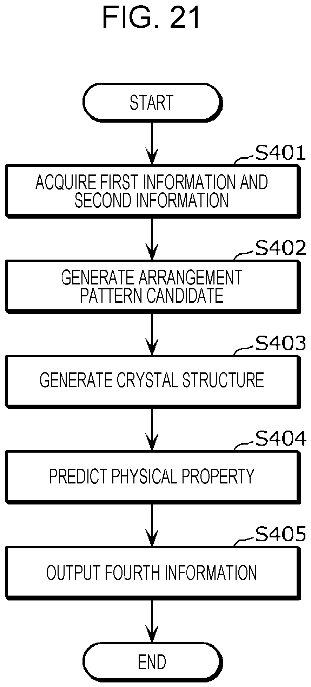

Operation of the information processing system 200 (i.e., information processing method) according to Embodiment 3 is described below. Fig. 21 is a flowchart illustrating an operation example of the information processing system 200 according to Embodiment 3.

(Step S401)

The first acquisition unit 11 acquires first information. In Embodiment 3, three-dimensional structure stored in a first storage unit 4 is read out, and a user inputs (selects) the first information by using the input unit 2 while seeing a first image displayed on a display unit 3, as in Embodiment 1. In this way, the first information is acquired by a first acquisition unit 11. The second acquisition unit 12 acquires the second information. The second information is similar to that in Embodiment 1.

(Step S402)

The generation unit 13 performs processing for generating an arrangement pattern candidate on the basis of the acquired first information and second information, as in Embodiment 1.

(Step S403)

The generation unit 13 performs processing for generating a crystal structure for each arrangement pattern candidate, as in Embodiment 1. In this example, it is assumed that the generation unit 13 generates a crystal structure for all arrangement pattern candidates in step S403.

(Step S404)

The generation unit 13 performs processing for predicting a physical property of the crystal structure on the basis of the generated crystal structure and the compound data read out from the third storage unit 6. For example, the generation unit 13 calculates convex hull energy as the physical property of the crystal structure. The convex hull energy is expressed by the following formula, for example, in a case where a composition of the crystal structure is "$CuInSe_2$".

[Math. 1]

$$Ehull(CuInSe_2) = Etot(CuInSe_2) - \frac{Etot(Cu_2Se)}{2} - \frac{Etot(In_2Se_3)}{2}$$

In the above formula, "Ehull (A)" represents convex hull energy of a crystal structure "A", and "Etot (A)" represents total energy of the crystal structure "A". The total energy of the crystal structure can be calculated by ab initio calculation or a prediction model trained by machine learning. Note that it is suggested that there is synthesizability as long as convex hull energy is 0.1 eV or less, as described, for example, in a thesis of Wenhao et al. (S. Wenhao, et al. "The thermodynamic scale of inorganic crystalline metastability. "Science advances 2.11 (2016) : e1600225.). That is, the convex hull energy is an index of synthesizability of a crystal structure.

(Step S405)

The output unit 14 performs processing for outputting the fourth information generated by the generation unit 13. In this example, the output unit 14 outputs the fourth information by displaying a third image indicative of the fourth information generated by the generation unit 13 on the display unit 3.

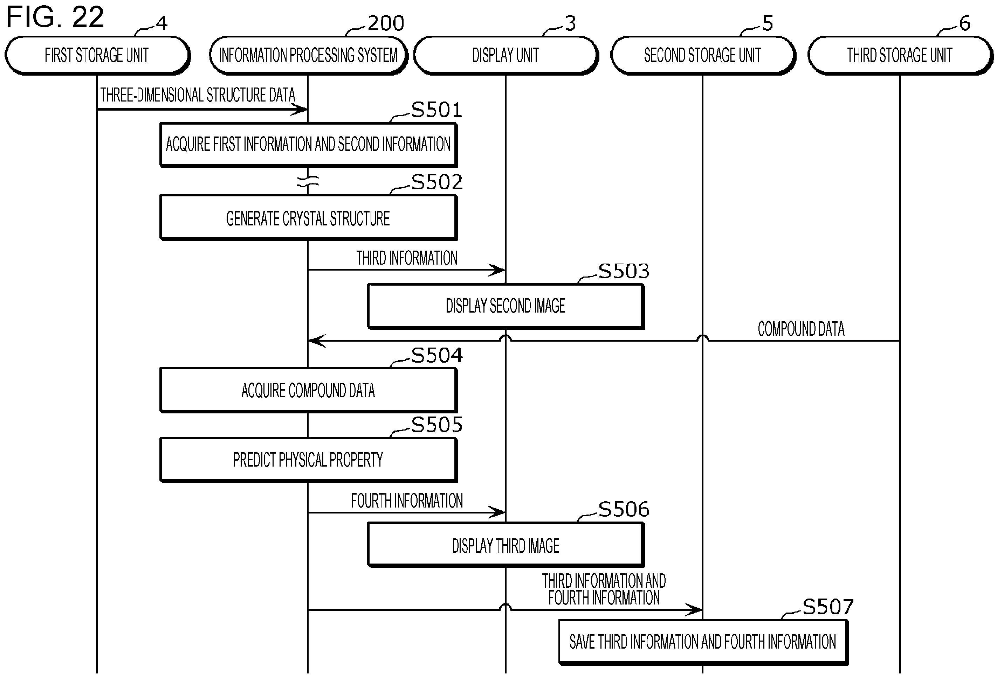

An operation example of the information processing system 200 and the display unit 3, the first storage unit 4, and the second storage unit 5 according to Embodiment 3 is described below with reference to the drawings. Fig. 22 is a sequence diagram illustrating an operation example of the information processing system 200 and the display unit 3, the first storage unit 4, and the second storage unit 5 according to Embodiment 3.

(Step S501)

The first acquisition unit 11 of the information processing system 200 acquires the first information. In this example, three-dimensional structure data stored in the first storage unit 4 is read out, and a user inputs (selects) the first information by using the input unit 2 while seeing the first image displayed on the display unit 3. In this way, the first information is acquired by the first acquisition unit 11. The second acquisition unit 12 of the information processing system 200 acquires the second information. In this example, the user inputs (selects) the second information by using the input unit 2 while seeing the first image displayed on the display unit 3. In this way, the second information is acquired by the second acquisition unit 12.

(Step S502)

The generation unit 13 of the information processing system 200 performs processing for generating a crystal structure for each arrangement pattern candidate. Note that between step S501 and step S502, processing identical to step S202 (see Fig. 17) is performed.

(Step S503)

The display unit 3 displays a second image indicative of third information output from the output unit 14 of the information processing system 200. The display unit 3 may include the display control unit 30. The display unit 3 including the display control unit 30 may be referred to as a display unit 3A. The output unit 14 may display the second image indicative of the third information on the display unit 3A.

(Step S504)

The first acquisition unit 11 (or the second acquisition unit 12) of the information processing system 200 acquires compound data by reading out the compound data stored in the third storage unit 6.

(Step S505)

The generation unit 13 of the information processing system 200 predicts a physical property of the crystal structure on the basis of the generated crystal structure and the compound data read out from the third storage unit 6.

(Step S506)

The display unit 3 displays a third image indicative of the fourth information output from the output unit 14 of the information processing system 200. The output unit 14 may display the third image indicative of the fourth information on the display unit 3A.

(Step S507)

When the user selects a crystal structure to be saved while seeing the third image displayed on the display unit 3, the information processing system 200 gives the third information indicative of the selected crystal structure and the fourth information corresponding to the selected crystal structure to the second storage unit 5. In this way, the second storage unit 5 stores therein the third information indicative of the crystal structure selected by the user and the fourth information corresponding to the selected crystal structure.

[Use Examples]

Use examples of the information processing system 200 according to Embodiment 3 are listed below. In description of a second use example and a third use example below, description of points identical to a first use example is omitted.

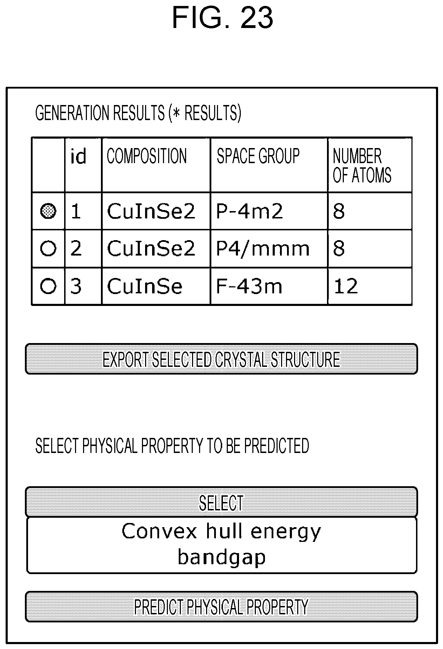

Fig. 23 illustrates an example of the second image displayed on the display unit 3 in the first use example of Embodiment 3. The second image is displayed on the display unit 3 after a user selects an execution icon in the first image and the generation unit 13 generates the third information indicative of a crystal structure. The second image includes a table showing a list of crystal structures generated by the generation unit 13 and an execution icon "EXPORT SELECTED CRYSTAL STRUCTURE", as in Embodiment 1. In the first use example, the second image further includes a physical property selection region for selecting a physical property of a crystal structure to be predicted and a prediction execution icon "PREDICT PHYSICAL PROPERTY".

In the physical property selection region, a physical property of a crystal structure that can be selected by the user is displayed. Note that although the user can select any one of "convex hull energy" and "bandgap" in the example illustrated in Fig. 23, another physical property of a crystal structure may be selectable.

In a case where the user desires prediction of a physical property of a crystal structure, the user selects a physical property of a crystal structure to be predicted in the physical property selection region and then selects the prediction execution icon. As a result, the generation unit 13 performs processing for predicting the physical property of the crystal structure. On the other hand, in a case where the user thinks prediction of a physical property of a crystal structure is unnecessary, the user selects a crystal structure to be saved and selects the execution icon. In this case, the user can save the selected crystal structure in the second storage unit 5 after confirming the crystal structure without obtaining a prediction result of a physical property of the crystal structure.

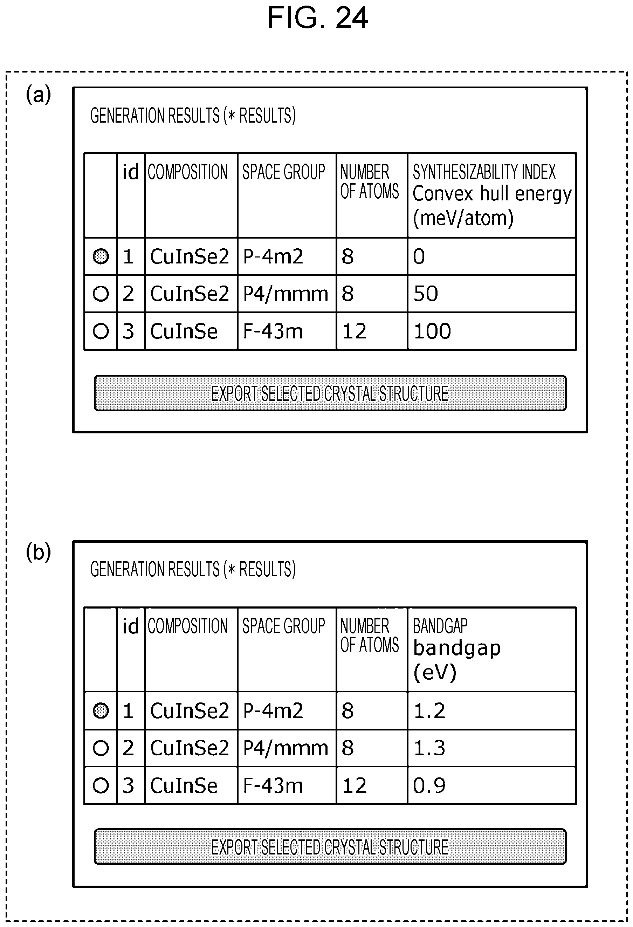

Fig. 24 illustrates an example of the third image displayed on the display unit 3 according to the first use example of Embodiment 3. The third image is displayed on the display unit 3 after the user selects the prediction execution icon in the second image and the generation unit 13 generates the fourth information concerning a physical property of a crystal structure. The third image includes a table showing a list of crystal structures generated by the generation unit 13 and physical properties of the crystal structures predicted by the generation unit 13 and an execution icon "EXPORT SELECTED CRYSTAL STRUCTURE".

The third image illustrated in Fig. 24(a) is an image displayed in a case where the user selects "convex hull energy" in the physical property selection region. In the third image illustrated in Fig. 24(a), the table includes a "synthesizability index" column, which shows convex hull energy of a crystal structure. Note that the smaller the convex hull energy of a crystal structure is, the higher the synthesizability is.

The third image illustrated in Fig. 24(b) is an image displayed in a case where the user selects "bandgap" in the physical property selection region. In the third image illustrated in Fig. 24(a), the table includes a "bandgap" column, which shows a bandgap of a crystal structure.

The user selects a crystal structure to be saved and selects the execution icon. As a result, the user can save the third information indicative of the selected crystal structure and the fourth information concerning a physical property of the selected crystal structure in the second storage unit 5 after confirming the selected crystal structure.

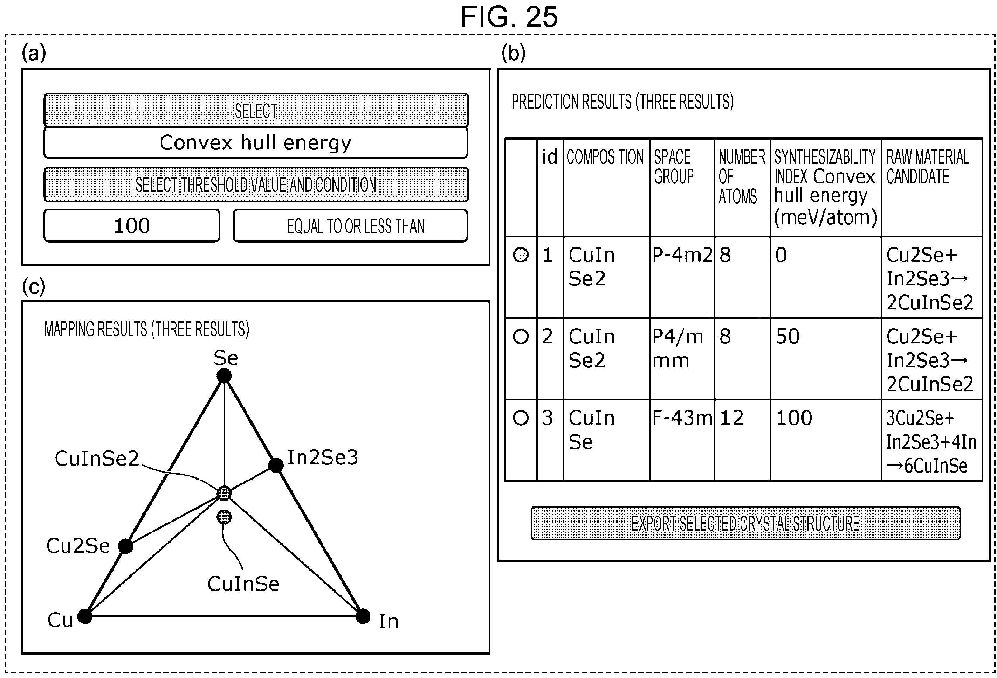

Fig. 25 illustrates an image displayed on the display unit 3 in the second use example of Embodiment 3. Fig. 25(a) illustrates a part of a first image displayed next on the display unit 3 when the user selects a physical property of a crystal structure in the physical property selection region in the first image. In the example illustrated in Fig. 25(a), the user selects "convex hull energy" as a physical property of a crystal structure. As a result, an execution icon "SELECT THRESHOLD VALUE AND CONDITION", a textbox for designating a threshold value, and a pull-down menu for designating a condition are displayed on the display unit 3.

In the example illustrated in Fig. 25(a), the user inputs "100" in the textbox and selects "EQUAL TO OR LESS THAN" in the pull-down menu. In this case, when the user selects the execution icon, the generation unit 13 performs prediction so as to narrow generated crystal structures down to a crystal structure whose convex hull energy is equal to or less than 100 meV/atm. That is, the generation unit 13 (in a step of generating the third information) generates the third information only for a crystal structure having a predetermined physical property (in this example, convex hull energy equal to or less than 100 meV/atm) on the basis of calculated fourth information (in this example, convex hull energy).

Fig. 25(b) illustrates an example of the third image displayed on the display unit 3. As illustrated in Fig. 25(b), the third image further includes a "raw material candidate" column showing a candidate for a raw material for generating a crystal structure in the table, unlike the first use example. Note that the candidate for the raw material shown in the "raw material candidate" column is a raw material that has a possibility of generation of a crystal structure, and a corresponding crystal structure cannot always be generated when the raw material is synthesized.

Fig. 25(c) illustrates an example of the third image displayed on the display unit 3. The image illustrated in Fig. 25(c) may be displayed on the display unit 3 concurrently with the image illustrated in Fig. 25(b) or may be displayed on the display unit 3 separately from the image illustrated in Fig. 25(b). The third image illustrated in Fig. 25(c) includes a mapping region. In the mapping region, a mapping result indicative of the number of crystal structures that satisfy the designated threshold value and condition and a map whose vertexes are elements (atoms) constituting the crystal structures and showing, on a line, compounds made of up the elements are displayed. In the map, a composition of a crystal structure and a composition that exhibits a phase thermodynamically coexisting with the crystal structure are displayed. In the map, compositions whose convex hull energy is zero, that is, compositions that exhibit a thermodynamically stable phase are connected by a straight line.

In the map illustrated in Fig. 25(c), a crystal structure whose composition is "$CuInSe_2$" is located on a line. Accordingly, the user can grasp that synthesizability of this crystal structure is relatively high. On the other hand, in the map illustrated in Fig. 25(c), a crystal structure whose composition is "CuInSe" is not located on a line. Accordingly, the user can grasp that synthesizability of this crystal structure is relatively low.

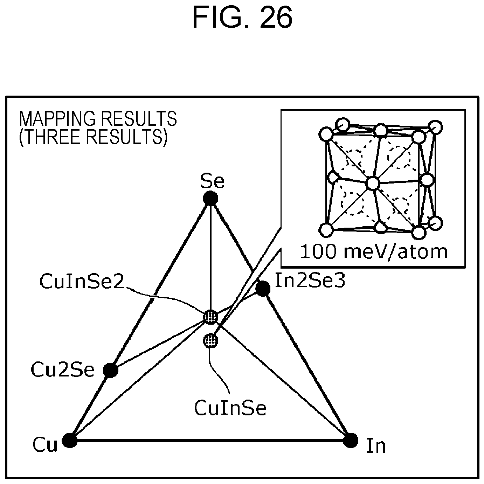

Fig. 26 illustrates another image displayed on the display unit 3 in the second use example of Embodiment 3. The image illustrated in Fig. 26 is displayed on the display unit 3 in a case where the user selects a composition of a crystal structure on the map in the image illustrated in Fig. 25(c), and includes an image of the crystal structure and a physical property (in this example, convex hull energy) of the crystal structure. The example illustrated in Fig. 26 illustrates an image displayed in a case where the user selects the crystal structure composition "CuInSe".

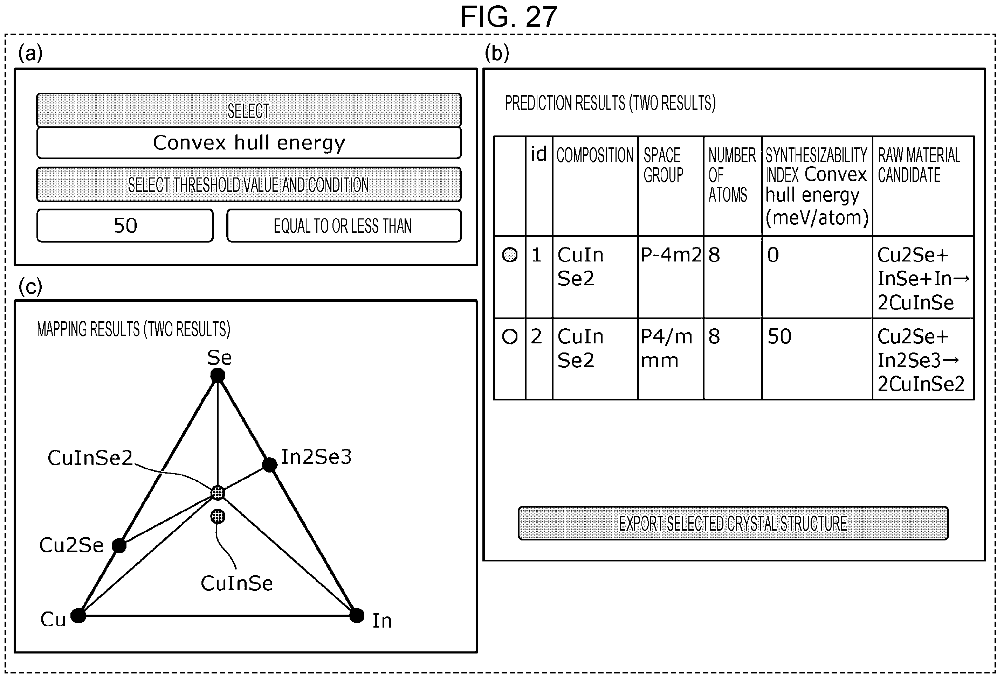

Fig. 27 illustrates still another image displayed on the display unit 3 in the second use example of Embodiment 3. The image illustrated in Fig. 27(a) includes an execution icon "SELECT THRESHOLD VALUE AND CONDITION", a textbox for designating a threshold value, and a pull-down menu for designating a condition, as in the image illustrated in Fig. 25(a). In the example illustrated in Fig. 27(a), the user inputs "50" in the textbox and selects "EQUAL TO OR LESS THAN" in the pull-down menu. In this case, when the user selects the execution icon, the generation unit 13 performs prediction so as to narrow generated crystal structures down to a crystal structure whose convex hull energy is equal to or less than 50 meV/atm.

The image illustrated in Fig. 27(b) is an image similar to the image illustrated in Fig. 25(b) and illustrates a processing result of the generation unit 13 under the threshold value and condition illustrated in Fig. 27(a). The image illustrated in Fig. 27(c) is an image similar to the image illustrated in Fig. 25(c) and illustrates a processing result of the generation unit 13 under the threshold value and condition illustrated in Fig. 27(a).

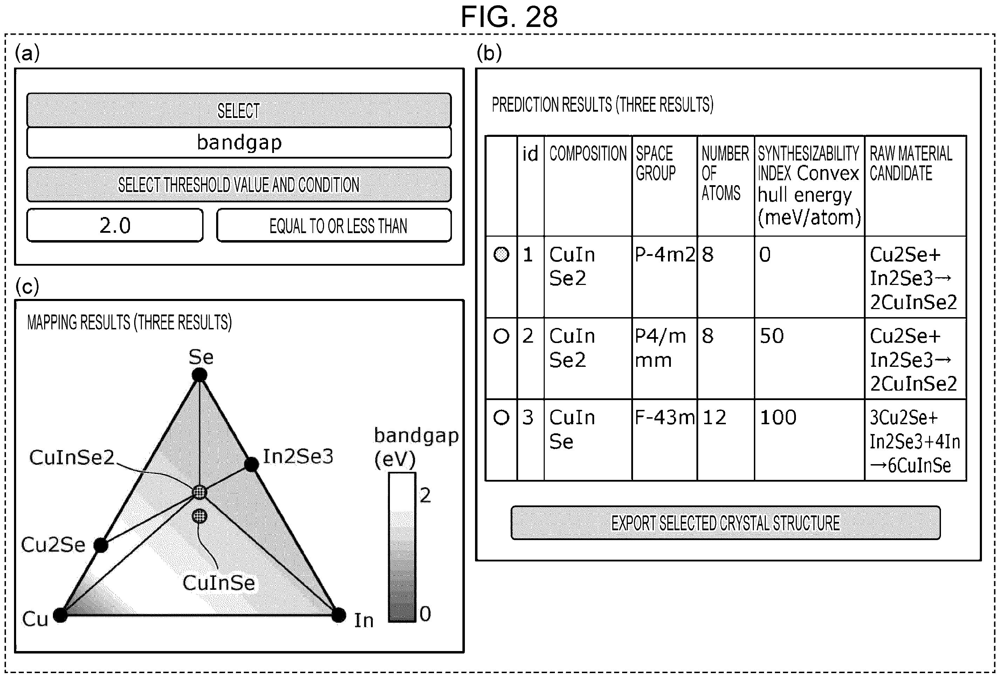

Fig. 28 illustrates an image displayed on the display unit 3 in a case where another physical property is selected in the second use example of Embodiment 3. In the example illustrated in Fig. 28(a), the user selects "bandgap" as a physical property of a crystal structure, inputs "2.0" in the textbox, and selects "EQUAL TO OR LESS THAN" in the pull-down menu. In this case, when the user selects the execution icon, the generation unit 13 performs prediction so as to narrow generated crystal structures down to a crystal structure whose bandgap is equal to or less than 2.0 eV.

Fig. 28(b) illustrates an image similar to the image illustrated in Fig. 25(b) and illustrates a processing result of the generation unit 13 under the threshold value and condition illustrated in Fig. 28(a). In the example illustrated in Fig. 28(b), the third image includes a "bandgap" column instead of the "convex hull energy" column.

Fig. 28(c) illustrates an image similar to the image illustrated in Fig. 25(c) and illustrates a processing result of the generation unit 13 under the threshold value and condition illustrated in Fig. 28(a). In the example illustrated in Fig. 28(c), a map showing magnitudes of bandgaps as shades is displayed in a mapping region of the third image.

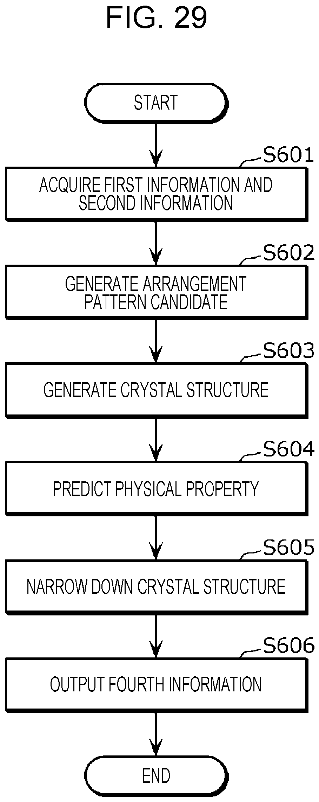

Another operation example of the information processing system 200 according to Embodiment 3, that is, an operation example in the first use example and the second use example is described below with reference to the drawings. Fig. 29 is a flowchart illustrating another operation example of the information processing system according to Embodiment 3.

(Step S601)

The first acquisition unit 11 acquires the first information. The second acquisition unit 12 acquires the second information. Step S601 is similar to step S401.

(Step S602)

The generation unit 13 performs processing for generating an arrangement pattern candidate on the basis of the acquired first information and second information, as in step S402.

(Step S603)

The generation unit 13 performs processing for generating a crystal structure for all arrangement pattern candidates, as in step S403.

(Step S604)

The generation unit 13 performs processing for predicting a physical property of the crystal structure on the basis of the generated crystal structure and the compound data read out from the third storage unit 6, as in step S404.

(Step S605)

The generation unit 13 generates the third information only for a crystal structure having a predetermined physical property, in other words, by narrowing the generated crystal structures down into a crystal structure having a predetermined physical property. The predetermined physical property is a threshold value and a condition (e.g., convex hull energy equal to or less than 100 meV/atm) designated by a user.

(Step S606)

The output unit 14 performs processing for outputting the fourth information generated by the generation unit 13, as in step S405. In this example, the fourth information includes the crystal structure found by narrowing-down in step S605.

As described above, in Embodiment 3, not only a crystal structure can be generated, but also a physical property of the generated crystal structure can be predicted and output. Therefore, in Embodiment 3, a user can efficiently search for an unknown material by referring to the physical property of the generated crystal structure.

(Modifications)

Although an information processing system (information processing method) according to one or more aspects of the present disclosure has been described above on the basis of the embodiments, the present disclosure is not limited to these embodiments. Various modifications of the above embodiments which a person skilled in the art can think of may be encompassed within the present disclosure without departing from the spirit of the present disclosure. Furthermore, combinations of constituent elements in different embodiments may also be encompassed within the present disclosure.

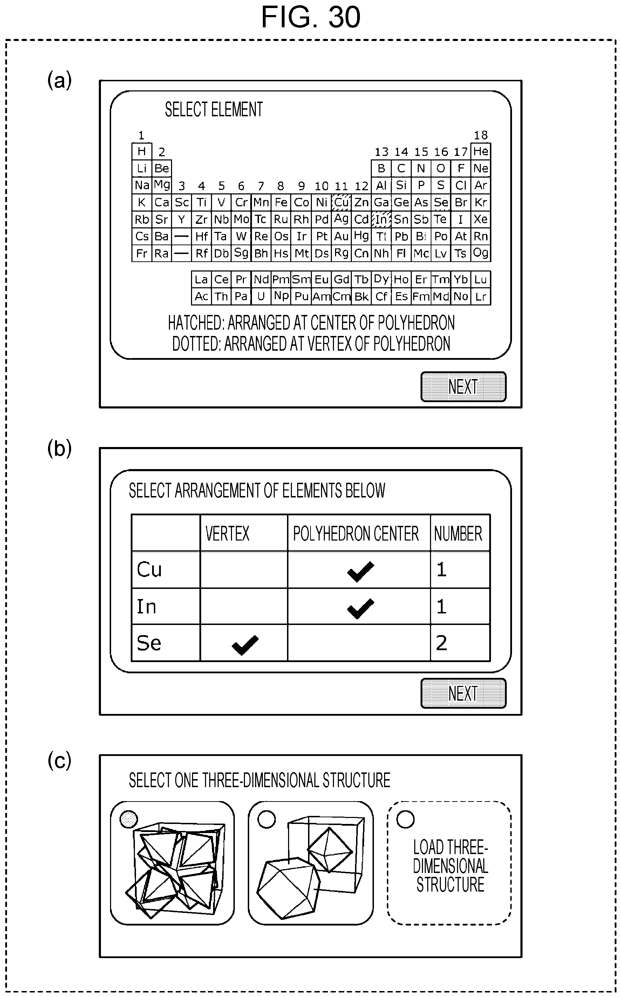

For example, in the above embodiments, the first image may be displayed on the display unit 3 in an order of the image illustrated in Fig. 30(a), the image illustrated in Fig. 30(b), and the image illustrated in Fig. 30(c). Fig. 30 illustrates an image displayed on the display unit 3 in a modification.

Fig. 30(a) illustrates an example of the first image initially displayed on the display unit 3. The first image illustrated in Fig. 30(a) includes an element selection region for selecting an element and an execution icon "NEXT". In the element selection region, a periodic table is displayed. The user selects an element (atom) contained in a desired material in the element selection region. For example, in a case where the user performs the operation of selecting an element one time, the element becomes an element disposed at a center of a polyhedron. On the other hand, in a case where the user performs the operation of selecting an element two times, the element becomes an element disposed at a vertex of the polyhedron. Then, when the user selects the execution icon, the second acquisition unit 12 (in a step of acquiring the second information) acquires, as the second information, material information concerning a composition of a material (in this example, atoms contained in the material).

Fig. 30(b) illustrates an example of the first image displayed next on the display unit 3. The first image illustrated in Fig. 30(b) is displayed on the display unit 3 in a case where the user selects the execution icon in the first image illustrated in Fig. 30(a). In the first image illustrated in Fig. 30(b), an arrangement designation region for designating an arrangement of elements (atoms) contained in a material and an execution icon "NEXT" are displayed. In the arrangement designation region, a table showing the number of elements in a polyhedron and positions (a vertex or a center) of the elements in the polyhedron is displayed.

In a case where the first image illustrated in Fig. 30(b) is displayed on the display unit 3 after the first image illustrated in Fig. 30(a) is displayed, positions of elements are already designated in the arrangement designation region. Accordingly, the user designates the number of elements in a polyhedron and selects the execution icon. In this case, the second acquisition unit 12 (in the step of acquiring the second information) acquires second information concerning an arrangement of elements (atoms) arranged in polyhedra.

Fig. 30(c) illustrates an example of the first image displayed next on the display unit 3. The first image illustrated in Fig. 30 is displayed on the display unit 3 in a case where the user selects the execution icon in the first image illustrated in Fig. 30(b). The first image illustrated in Fig. 30(c) includes a three-dimensional structure selection region, as in the first image illustrated in Fig. 8 of Embodiment 1.

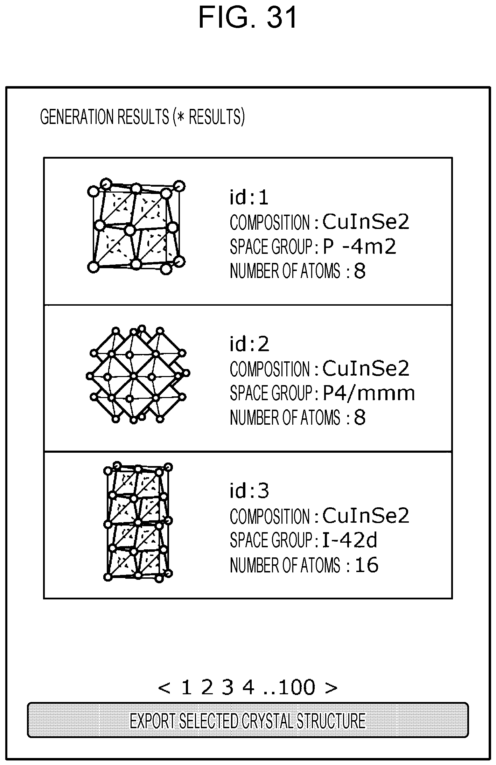

Fig. 31 illustrates an image displayed on the display unit 3 in a modification. Fig. 31 illustrates the second image, which is displayed on the display unit 3 after the third information indicative of a crystal structure is generated by the generation unit 13. The second image includes a list of crystal structures generated by the generation unit 13 and an execution icon "EXPORT SELECTED CRYSTAL STRUCTURE".

For example, although the information processing system displays the first to third images on the display unit 3 in the above embodiments, this is not restrictive. For example, the information processing system may output information included in the first to third images without displaying the first to third images themselves on the display unit 3.

Although the first acquisition unit 11 of the information processing system acquires the first information input by the user by using the input unit 2 in the above embodiments, this is not restrictive. For example, the first acquisition unit 11 may acquire the first information by reading out information stored in the first storage unit 4 without receiving user's input.

Although the information processing system generates a crystal structure while using a three-dimensional structure or a base crystal structure as the first information in the above embodiments, this is not restrictive. For example, the information processing system may generate a crystal structure while using polyhedra as the first information. In this case, the generation unit 13 performs processing for generating a crystal structure, for example, after generating a three-dimensional structure from the polyhedra used as the first information.

A method for generating a three-dimensional structure from polyhedra can be realized by expressing a three-dimensional structure as a numerical sequence or a graph. Note that the "numerical sequence" as used herein includes not only a numeral, but also a character, such as an alphabet, replacing a numeral.

Hereinafter, a numerical sequence expressing a three-dimensional structure is sometimes referred to as an "inorganic gene". Examples of the inorganic gene include a polychoron code proposed by K. Nishio et al., Systre Key or D-Symbol proposed by O. Delgado-Friedrichs et al., and CRYSTAL-SELFIES proposed by M. Krenn et al., which is application of SELFIE that can express a molecular structure as an alphabet sequence to a three-dimensional structure. In other words, the inorganic gene is, for example, a polychoron code that can be converted into a three-dimensional structure.

For example, a polychoron code of a zeolite A (LTA) structure is expressed as a numerical sequence "OHG$^4$ (HG)$^4$H". Note that "O", "H", and "G" in the numerical sequence are called polyhedron codes and are numerical sequences decided on the basis of input polyhedra. For example, "O" means a truncated octahedron and is expressed as a numerical sequence "46$^4$(46)$^4$4". Furthermore, for example, "H" means a cube and is expressed as a numerical sequence "46". Furthermore, for example, "G" means a truncated cuboctahedron and is expressed as a numerical sequence "6(48)$^3$(64)$^6$ (84)$^3$6".

By rearranging the polychoron code, another three-dimensional structure made up of the same polyhedra can be generated. Furthermore, by changing a polyhedron code, any polyhedron can be expressed. By thus using an inorganic gene represented by a polychoron code, a three-dimensional structure can be exhaustively generated from information on polyhedra.

A method for generating a three-dimensional structure from polyhedra can be realized by expressing a three-dimensional structure as a periodic graph. For example, a polyhedron can be converted into a polyhedron graph by connecting each vertex site and a center site of the polyhedron. For example, a periodic graph can be generated by coupling vertex nodes in the polyhedron graphs thus obtained. Then, the periodic graph can be converted into a three-dimensional structure, for example, by using the method shown in the Kotani-Sunada theory (Kotani-Sunada, 2000, Trans. Amer. Mat).

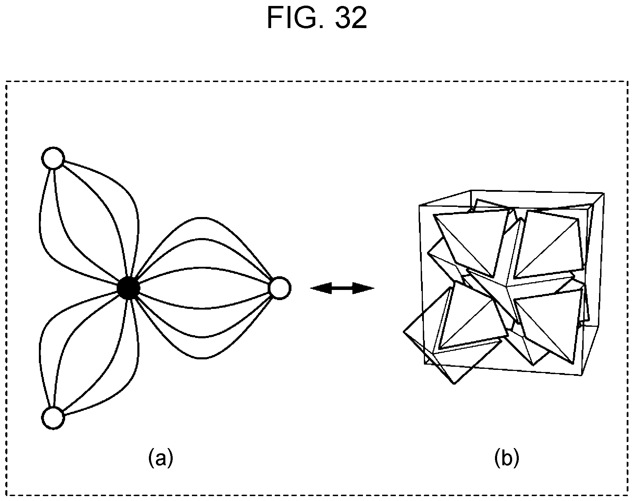

Fig. 32 illustrates a specific example of a case where a periodic graph is converted into a three-dimensional structure. The three-dimensional structure (fcc-type structure) illustrated in Fig. 32(b) is generated by converting the periodic graph illustrated in Fig. 32(a). By thus using a periodic graph, a three-dimensional structure can be exhaustively generated from information on polyhedra.

Although the first storage unit 4 and the second storage unit 5 are realized by different recording media in Embodiments 1 and 2, this is not restrictive. For example, the first storage unit 4 and the second storage unit 5 may be realized by the same recording medium. Similarly, although the first storage unit 4, the second storage unit 5, and the third storage unit 6 are realized by different recording media in Embodiment 3, this is not restrictive. For example, the first storage unit 4, the second storage unit 5, and the third storage unit 6 may be realized by the same recording medium.

Although the first acquisition unit 11 and the second acquisition unit 12 are different acquisition units in the above embodiments, the first acquisition unit 11 and the second acquisition unit 12 may be realized by the same acquisition unit.

Although each of the information processing systems 100 and 200 includes the first acquisition unit 11, the second acquisition unit 12, the generation unit 13, and the output unit 14 in the above embodiments, this is not restrictive. For example, the information processing system 100 may include the display control unit 30 and the display unit 3, as indicated by "100A" in Fig. 5. Similarly, the information processing system 200 may include the display control unit 30 and the display unit 3.

Although the first image is configured as a single image to receive input of the first information and the second information in the above embodiments, an image for receiving input of the first information and an image for receiving input of the second information may be separately displayed sequentially on the display unit 3 as the first image. In this case, the former image may be displayed on the display unit 3 earlier or the latter image may be displayed on the display unit 3 earlier.

For example, in the example illustrated in Fig. 30, the first image includes a first input image (the image illustrated in Fig. 30(c)) for receiving input of the first information and a second input image (the images illustrated in Fig. 30(a) and (b)) for receiving input of the second information. In the example illustrated in Fig. 30, the display control unit 30 causes the second input image to be displayed on the display unit 3 and causes the first input image to be displayed on the display unit 3 on the basis of the input second information.

Note that in the above embodiments, each constituent element may be realized by dedicated hardware or may be realized by execution of a software program suitable for the constituent element. Each constituent element may be realized by reading out and executing a software program recorded in a recording medium such as a hard disk or a semiconductor memory by a program execution unit such as a central processing unit (CPU) or a processor.

Note that the following cases are also encompassed within the present disclosure.

(1) At least one of the apparatuses described above is specifically a computer system that includes a microprocessor, a Read Only Memory (ROM), a Random Access Memory (RAM), a hard disk unit, a display unit, a keyboard, a mouse, and the like. A computer program is stored in the RAM or the hard disk unit. The microprocessor operates in accordance with the computer program, and thus the at least one of the apparatuses accomplishes a function thereof. The computer program is a combination of command codes indicating a command given to a computer for accomplishment of a predetermined function.

(2) Part of or all of constituent elements that constitute at least one of the apparatuses may include a single system large scale integration (LSI). The system LSI is a super-multi-function LSI produced by integrating constituents on a single chip and is specifically a computer system including a microprocessor, a ROM, a RAM, and the like. A computer program is stored in the RAM. The microprocessor operates in accordance with the computer program, and thus the system LSI accomplishes a function thereof.

(3) Part of or all of constituent elements that constitute at least one of the apparatuses may include an IC card that can be detachably attached to the apparatus or a stand-alone module. The IC card or the module is a computer system that includes a microprocessor, a ROM, a RAM, and the like. The IC card or the module may include the super-multi-function LSI. The microprocessor operates in accordance with a computer program, and thus the IC card or the module accomplishes a function thereof. The IC card or the module may have tamper resistance.

(4) The present disclosure may be the methods described above. The present disclosure may be a computer program for causing a computer to realize these methods or may be a digital signal represented by the computer program.

The present disclosure may be a computer-readable recording medium, such as a flexible disc, a hard disk, a Compact Disc (CD)-ROM, a DVD, a DVD-ROM, a DVD-RAM, a Blu-ray (Registered Trademark) (BD) Disc, or a semiconductor memory, on which the computer program or the digital signal is recorded. The present disclosure may be the digital signal recorded on such a recording medium.

The present disclosure may be the computer program or the digital signal transmitted over an electric communication line, a wireless or wired communication line, a network represented by the Internet, data broadcasting, or the like.

The program or the digital signal may be executed by another independent computer system by transporting the program or the digital signal on the recording medium or transporting the program or the digital signal over the network or the like.

(Other Remarks)

A modification of the embodiments of the present disclosure may be as follows.

An information processing method being performed by one or more processors configured to execute instructions stored in one or more memories, the information processing method including:

acquiring first information concerning polyhedra,
second information concerning first atoms,
generating third information indicative of a crystal structure on the basis of the first information and the second information, the crystal structure having a three-dimensional structure in which the polyhedra are arranged and including the first atoms each of which includes one or more atoms, and
outputting the generated third information.

Industrial Applicability

The present disclosure is useful for searching for an unknown material.

Reference Signs List

11 first acquisition unit
12 second acquisition unit
13 generation unit
14 output unit
2 input unit
3 display unit

30 display controller
4 first storage unit
5 second storage unit
6 third storage unit
100,200 information processing system
100A,200A information processing system

**Claims**

1. An information processing method performed by a computer, the information processing method comprising:

   acquiring first information concerning polyhedra;
   acquiring second information concerning atoms arranged in the polyhedra;
   generating third information indicative of a crystal structure that can be taken in a case where the atoms are arranged in a three-dimensional structure in which the polyhedra are arranged on a basis of the first information and the second information; and
   outputting the generated third information.

2. The information processing method according to claim 1, wherein
   the three-dimensional structure is a structure in which the polyhedra are arranged without any gaps.

3. The information processing method according to claim 2, wherein
   in the acquiring the first information, information concerning the three-dimensional structure in which the polyhedra are arranged without any gaps is acquired as the first information.

4. The information processing method according to claim 3, wherein
   the information concerning the three-dimensional structure includes at least one of information indicative of the three-dimensional structure, information indicative of a numerical sequence including a numeral or a character representing the three-dimensional structure, or information indicative of a periodic graph representing the three-dimensional structure.

5. The information processing method according to any one of claims 1 to 4, wherein
   in the generating the third information, the third information indicative of the crystal structure is generated by using an arrangement pattern of the atoms that can be taken in the crystal structure.

6. The information processing method according to claim 5, wherein
   the arrangement pattern indicates that the atoms are arranged at vertexes of the polyhedra and/or insides of the polyhedra.

7. The information processing method according to any one of claims 1 to 6, wherein

   in the acquiring the second information, material information concerning a material containing the atoms arranged in the polyhedra is acquired as the second information, and
   in the generating the third information, the third information indicative of the crystal structure which the material can take is generated on a basis of the material information.

8. The information processing method according to claim 7, wherein

   in the acquiring the second information, element information indicative of a kind of each of the atoms is further acquired as the material information, and
   in the generating the third information, the third information indicative of the crystal structure in which the atoms are arranged so that each kind indicated by the element information includes one or more atoms is generated.

9. The information processing method according to claim 7 or 8, wherein

   in the acquiring the second information, composition information concerning a composition of the material is further acquired as the material information, and

in the generating the third information, the third information indicative of the crystal structure having the composition indicated by the composition information is generated.

10. The information processing method according to any one of claims 7 to 9, wherein

in the acquiring the second information, atom number information indicative of the number of atoms arranged in the polyhedra is further acquired as the material information, and
in the generating the third information, the third information indicative of the crystal structure in which as many atoms as the number indicated by the atom number information are arranged is generated.

11. The information processing method according to any one of claims 1 to 10, wherein

in the acquiring the first information, crystal structure information indicative of a base crystal structure, which is a base of the crystal structure, is acquired as the first information, and
in the acquiring the third information, the third information indicative of the crystal structure that can be taken in a case where the atoms are arranged in the base crystal structure indicated by the crystal structure information is generated.

12. The information processing method according to claim 11, wherein
in the generating the third information, positions of vertexes of the polyhedra and positions of insides of the polyhedra in which the atoms are arranged are determined on a basis of the base crystal structure indicated by the crystal structure information.

13. The information processing method according to any one of claims 1 to 12, further comprising calculating and outputting fourth information concerning a physical property of the crystal structure indicated by the third information by using at least one of ab initio calculation or a prediction model trained by machine learning.

14. The information processing method according to claim 13, wherein
in the generating the third information, the third information is output only for the crystal structure having a predetermined physical property on a basis of the calculated fourth information.

15. An information processing system comprising:

a display that displays a first image for receiving input of first information concerning polyhedra and second information concerning atoms arranged in the polyhedra; and
a display controller that causes a second image to be displayed on the display, the second image showing third information indicative of a crystal structure that can be taken in a case where the atoms are arranged in a three-dimensional structure in which the polyhedra are arranged, the third information being generated on a basis of the input first information and second information.

16. The information processing system according to claim 15, wherein

the first image includes a first input image for receiving input of the first information and a second input image for receiving input of the second information, and
the display controller causes the second input image to be displayed on the display, and causes the first input image to be displayed on the display on a basis of the input second information.

17. A program causing a computer to perform processing comprising:

acquiring first information concerning polyhedra;
acquiring second information concerning atoms arranged in the polyhedra;
generating third information indicative of a crystal structure that can be taken in a case where the atoms are arranged in a three-dimensional structure in which the polyhedra are arranged on a basis of the first information and the second information; and
outputting the generated third information.

## FIG. 1

(a)

(d)

(b)

(e)

(c)

# FIG. 2

## FIG. 3

(a)

(b)

(c)

(d)

## FIG. 4

(a)

(b)

FIG. 5

INPUT UNIT — 2

INFORMATION PROCESSING SYSTEM — 100

FIRST STORAGE UNIT — 4

FIRST ACQUISITION UNIT — 11

SECOND ACQUISITION UNIT — 12

GENERATION UNIT — 13

SECOND STORAGE UNIT — 5

OUTPUT UNIT — 14

— 100A

DISPLAY CONTROL UNIT — 30

DISPLAY UNIT — 3

FIG. 6

(a)

```
#@ info dim = 3
#@ info size = 2
#@ info transitivity = 1112
#@ info selfdual = no
#@ info signature = 2[3^4] + [3^8]
#@ info group = Fm-3m
#@ info minimal = yes
#@ info net = fcu
#@ name #1
<1.1:2 3:1 2,1 2,1 2,2:3 3,3 4,4>
```

(b)

# FIG. 7

```
#
data_NaCl
_symmetry_space_group_name_H-M 'P 1'
_cell_length_a 5.69169400
_cell_length_b 5.69169400
_cell_length_c 5.69169400
_cell_angle_alpha 90.00000000
_cell_angle_beta 90.00000000
_cell_angle_gamma 90.00000000
_symmetry_Int_Tables_number 1
_chemical_formula_structural NaCl
_chemical_formula_sum 'Na4 Cl4'
_cell_volume 184.38459333
_cell_formula_units_Z 4
loop_
_symmetry_equiv_pos_site_id
_symmetry_equiv_pos_as_xyz
1 'x, y, z'
loop_
_atom_site_type_symbol
_atom_site_label
_atom_site_symmetry_multiplicity
_atom_site_fract_x
_atom_site_fract_y
_atom_site_fract_z
_atom_site_occupancy
Na Na0 1 0.00000000 0.00000000 0.00000000 1
Na Na1 1 0.00000000 0.50000000 0.50000000 1
Na Na2 1 0.50000000 0.00000000 0.50000000 1
Na Na3 1 0.50000000 0.50000000 0.00000000 1
Cl Cl4 1 0.50000000 0.00000000 0.00000000 1
Cl Cl5 1 0.50000000 0.50000000 0.50000000 1
Cl Cl6 1 0.00000000 0.00000000 0.50000000 1
Cl Cl7 1 0.00000000 0.50000000 0.00000000 1
```

(a)                                        (b)

# FIG. 8

(a)

SELECT ONE THREE-DIMENSIONAL STRUCTURE

LOAD THREE-DIMENSIONAL STRUCTURE

DESIGNATE ELEMENT ARRANGED
AT VERTEXES AND POLYHEDRON CENTERS

VERTEXES $\quad$ | Se |

POLYHEDRON CENTERS $\quad$ | Cu In |

GENERATE

(b)

GENERATION RESULTS (* RESULTS)

|  | id | COMPOSITION | SPACE GROUP | NUMBER OF ATOMS |
|---|---|---|---|---|
| ⊙ | 1 | CuInSe2 | P-4m2 | 8 |
| ○ | 2 | CuInSe2 | P4/mmm | 8 |
| ○ | 3 | CuInSe | F-43m | 12 |

EXPORT SELECTED CRYSTAL STRUCTURE

EP 4 503 042 A1

# FIG. 9

(a)

CRYSTAL STRUCTURE OF id:1

SAVE IMAGE

(b)

CRYSTAL STRUCTURE OF id:2

SAVE IMAGE

(c)

CRYSTAL STRUCTURE OF id:3

SAVE IMAGE

(d)

SAVING FORMAT

SELECT

.cif
.vasp

SAVE

EP 4 503 042 A1

# FIG. 10

(a)

SELECT ONE THREE-DIMENSIONAL STRUCTURE

LOAD THREE-DIMENSIONAL STRUCTURE

DESIGNATE ELEMENT ARRANGED
AT VERTEXES AND POLYHEDRON CENTERS

VERTEXES

| Se |

POLYHEDRON CENTERS

| Cu In |

(OPTION) INPUT DESIRED COMPOSITION

| CuInSe2 |

(OPTION) INPUT MAXIMUM NUMBER OF ATOMS
IN UNIT CELL

| 16 |

GENERATE

(b)

GENERATION RESULTS (* RESULTS)

| | id | COMPOSITION | SPACE GROUP | NUMBER OF ATOMS |
|---|----|-------------|-------------|------------------|
| ◉ | 1 | CuInSe2 | P-4m2 | 8 |
| ○ | 2 | CuInSe2 | P4/mmm | 8 |
| ○ | 3 | CuInSe2 | I-42d | 16 |

< 1 2 3 4 ..100 >

EXPORT SELECTED CRYSTAL STRUCTURE

# FIG. 11

START

S101
ACQUIRE FIRST INFORMATION AND
SECOND INFORMATION

S102
GENERATE ARRANGEMENT
PATTERN CANDIDATE

S103
GENERATE CRYSTAL STRUCTURE

S104
IS THERE
ANOTHER ARRANGEMENT PATTERN
CANDIDATE?

Yes

No

S105
OUTPUT THIRD INFORMATION

END

## FIG. 12

| | VERTEX SITE | | | | TETRAHEDRON CENTER SITE | | | | | | | | OCTAHEDRON CENTER SITE | | | | COMPOSITION |
|---|---|---|---|---|---|---|---|---|---|---|---|---|---|---|---|---|---|
| | X1 | X2 | X3 | X4 | T1 | T2 | T3 | T4 | T5 | T6 | T7 | T8 | O1 | O2 | O3 | O4 | |
| ARRANGEMENT PATTERN CANDIDATE 1 | Se | Se | Se | Se | Cu | — | Cu | — | — | In | — | In | — | — | — | — | CuInSe2 |
| ARRANGEMENT PATTERN CANDIDATE 2 | Se | Se | Se | Se | — | — | Cu | Cu | In | In | — | — | — | — | — | — | CuInSe2 |
| ARRANGEMENT PATTERN CANDIDATE 3 | Se | Se | Se | Se | Cu | Cu | — | — | — | — | In | In | — | — | — | — | CuInSe2 |
| ARRANGEMENT PATTERN CANDIDATE 4 | Se | Se | Se | Se | In | Cu | Cu | In | Cu | In | In | Cu | — | — | — | — | CuInSe |
| ARRANGEMENT PATTERN CANDIDATE 5 | Se | Se | Se | Se | Cu | Cu | — | — | In | In | — | — | — | — | — | — | CuInSe2 |
| ARRANGEMENT PATTERN CANDIDATE 6 | Se | Se | Se | Se | Cu | — | — | — | In | — | — | — | — | — | — | — | CuInSe4 |
| ARRANGEMENT PATTERN CANDIDATE 7 | Se | Se | Se | Se | Cu | In | — | — | In | In | — | — | — | — | — | — | CuIn3Se4 |
| ARRANGEMENT PATTERN CANDIDATE 8 | Se | Se | Se | Se | — | — | — | — | — | — | — | — | Cu | In | Cu | In | CuInSe2 |
| ... | | | | | | | | | | | | | | | | | |

EP 4 503 042 A1

# FIG. 13

(a)  (b)  (c)

EP 4 503 042 A1

# FIG. 14

| | ... | TETRAHEDRON CENTER SITE | | | | | | | | ... | COMPOSITION |
|---|---|---|---|---|---|---|---|---|---|---|---|
| | | T1 | T2 | T3 | T4 | T5 | T6 | T7 | T8 | | |
| ARRANGEMENT PATTERN CANDIDATE 1 | | Cu | Cu | In | In | Cu | Cu | In | In | | CuInSe2 |
| ARRANGEMENT PATTERN CANDIDATE 2 | | In | In | Cu | Cu | In | In | Cu | Cu | | CuInSe2 |
| ARRANGEMENT PATTERN CANDIDATE 3 | | In | In | Cu | Cu | Cu | Cu | In | In | | CuInSe2 |
| ARRANGEMENT PATTERN CANDIDATE 4 | | Cu | Cu | In | In | In | In | Cu | Cu | | CuInSe2 |
| ARRANGEMENT PATTERN CANDIDATE 5 | | In | In | In | In | Cu | Cu | In | In | | CuIn3Se6 |
| ARRANGEMENT PATTERN CANDIDATE 6 | | — | — | In | In | Cu | Cu | In | In | | CuIn2Se4 |
| ARRANGEMENT PATTERN CANDIDATE 7 | | — | — | — | — | Cu | Cu | In | In | | CuIn3Se4 |
| ARRANGEMENT PATTERN CANDIDATE 8 | | — | — | — | — | — | — | — | — | | CuInSe2 |
| ... | | | | | | | | | | | |

## FIG. 15

## FIG. 16

## FIG. 17

FIRST STORAGE UNIT `4`  |  INFORMATION PROCESSING SYSTEM `100`  |  DISPLAY UNIT `3`  |  SECOND STORAGE UNIT `5`

THREE-DIMENSIONAL STRUCTURE DATA

**S201** ACQUIRE FIRST INFORMATION AND SECOND INFORMATION

**S202** GENERATE ARRANGEMENT PATTERN CANDIDATE

**S203** GENERATE CRYSTAL STRUCTURE

THIRD INFORMATION

**S204** DISPLAY SECOND IMAGE

THIRD INFORMATION

**S205** SAVE THIRD INFORMATION

# FIG. 18

SELECT ONE CRYSTAL STRUCTURE

LOAD CRYSTAL
STRUCTURE

DESIGNATE ELEMENT ARRANGED
AT VERTEXES AND POLYHEDRON CENTERS

VERTEXES

Se

POLYHEDRON CENTERS

Cu In

GENERATE

# FIG. 19

START

↓

S301

ACQUIRE FIRST INFORMATION
(CRYSTAL STRUCTURE) AND
SECOND INFORMATION

↓

S302

DETERMINE VERTEX SITES AND
CENTER SITES OF POLYHEDRA

↓

S303

GENERATE ARRANGEMENT
PATTERN CANDIDATE

↓

S304

GENERATE CRYSTAL STRUCTURE

↓

S305

IS THERE
ANOTHER ARRANGEMENT PATTERN
CANDIDATE?

Yes

No

↓

S306

OUTPUT THIRD INFORMATION

↓

END

# FIG. 20

# FIG. 21

```
           ┌─────────────┐
           │    START    │
           └──────┬──────┘
                  │          ╭─S401
                  ▼
     ┌────────────────────────────┐
     │ ACQUIRE FIRST INFORMATION AND │
     │      SECOND INFORMATION      │
     └──────────────┬─────────────┘
                    │        ╭─S402
                    ▼
     ┌────────────────────────────┐
     │    GENERATE ARRANGEMENT     │
     │     PATTERN CANDIDATE       │
     └──────────────┬─────────────┘
                    │        ╭─S403
                    ▼
     ┌────────────────────────────┐
     │   GENERATE CRYSTAL STRUCTURE │
     └──────────────┬─────────────┘
                    │        ╭─S404
                    ▼
     ┌────────────────────────────┐
     │   PREDICT PHYSICAL PROPERTY  │
     └──────────────┬─────────────┘
                    │        ╭─S405
                    ▼
     ┌────────────────────────────┐
     │   OUTPUT FOURTH INFORMATION  │
     └──────────────┬─────────────┘
                    ▼
           ┌─────────────┐
           │     END     │
           └─────────────┘
```

FIG. 22

# FIG. 23

GENERATION RESULTS (* RESULTS)

|  | id | COMPOSITION | SPACE GROUP | NUMBER OF ATOMS |
|---|---|---|---|---|
| ◉ | 1 | CuInSe2 | P-4m2 | 8 |
| ○ | 2 | CuInSe2 | P4/mmm | 8 |
| ○ | 3 | CuInSe | F-43m | 12 |

EXPORT SELECTED CRYSTAL STRUCTURE

SELECT PHYSICAL PROPERTY TO BE PREDICTED

SELECT

Convex hull energy
bandgap

PREDICT PHYSICAL PROPERTY

# FIG. 24

(a)

GENERATION RESULTS (* RESULTS)

| | id | COMPOSITION | SPACE GROUP | NUMBER OF ATOMS | SYNTHESIZABILITY INDEX Convex hull energy (meV/atom) |
|---|---|---|---|---|---|
| ◉ | 1 | CuInSe2 | P-4m2 | 8 | 0 |
| ○ | 2 | CuInSe2 | P4/mmm | 8 | 50 |
| ○ | 3 | CuInSe | F-43m | 12 | 100 |

EXPORT SELECTED CRYSTAL STRUCTURE

(b)

GENERATION RESULTS (* RESULTS)

| | id | COMPOSITION | SPACE GROUP | NUMBER OF ATOMS | BANDGAP bandgap (eV) |
|---|---|---|---|---|---|
| ◉ | 1 | CuInSe2 | P-4m2 | 8 | 1.2 |
| ○ | 2 | CuInSe2 | P4/mmm | 8 | 1.3 |
| ○ | 3 | CuInSe | F-43m | 12 | 0.9 |

EXPORT SELECTED CRYSTAL STRUCTURE

# FIG. 25

(a)

SELECT

Convex hull energy

SELECT THRESHOLD VALUE AND CONDITION

| 100 | EQUAL TO OR LESS THAN |

(c)

MAPPING RESULTS (THREE RESULTS)

(b)

PREDICTION RESULTS (THREE RESULTS)

| | id | COMPOSITION | SPACE GROUP | NUMBER OF ATOMS | SYNTHESIZABILITY INDEX Convex hull energy (meV/atom) | RAW MATERIAL CANDIDATE |
|---|---|---|---|---|---|---|
| ◉ | 1 | CuIn Se2 | P-4m2 | 8 | 0 | Cu2Se+ In2Se3→ 2CuInSe2 |
| ○ | 2 | CuIn Se2 | P4/m mm | 8 | 50 | Cu2Se+ In2Se3→ 2CuInSe2 |
| ○ | 3 | CuIn Se | F-43m | 12 | 100 | 3Cu2Se+ In2Se3+4In →6CuInSe |

EXPORT SELECTED CRYSTAL STRUCTURE

# FIG. 26

# FIG. 27

(a)

| SELECT |
| --- |

| Convex hull energy |
| --- |

| SELECT THRESHOLD VALUE AND CONDITION |
| --- |

| 50 | EQUAL TO OR LESS THAN |
| --- | --- |

(b)

PREDICTION RESULTS (TWO RESULTS)

| | id | COMPOSITION | SPACE GROUP | NUMBER OF ATOMS | SYNTHESIZABILITY INDEX Convex hull energy (meV/atom) | RAW MATERIAL CANDIDATE |
| --- | --- | --- | --- | --- | --- | --- |
| ◉ | 1 | CuInSe2 | P-4m2 | 8 | 0 | Cu2Se+InSe+In→2CuInSe |
| ○ | 2 | CuInSe2 | P4/mmm | 8 | 50 | Cu2Se+In2Se3→2CuInSe2 |

| EXPORT SELECTED CRYSTAL STRUCTURE |
| --- |

(c)

MAPPING RESULTS (TWO RESULTS)

Se

CuInSe2

In2Se3

Cu2Se

CuInSe

Cu

In

EP 4 503 042 A1

# FIG. 28

**(a)**

SELECT

bandgap

SELECT THRESHOLD VALUE AND CONDITION

| 2.0 | EQUAL TO OR LESS THAN |

**(c)**

MAPPING RESULTS (THREE RESULTS)

**(b)**

PREDICTION RESULTS (THREE RESULTS)

| | id | COMPOSITION | SPACE GROUP | NUMBER OF ATOMS | SYNTHESIZABILITY INDEX Convex hull energy (meV/atom) | RAW MATERIAL CANDIDATE |
|---|---|---|---|---|---|---|
| ◉ | 1 | CuInSe2 | P-4m2 | 8 | 0 | Cu2Se+In2Se3→2CuInSe2 |
| ○ | 2 | CuInSe2 | P4/mmm | 8 | 50 | Cu2Se+In2Se3→2CuInSe2 |
| ○ | 3 | CuInSe | F-43m | 12 | 100 | 3Cu2Se+In2Se3+4In→6CuInSe |

EXPORT SELECTED CRYSTAL STRUCTURE

EP 4 503 042 A1

# FIG. 29

```
                    ┌──────────────┐
                    │    START     │
                    └──────┬───────┘
                           │
                           ▼              S601
        ┌─────────────────────────────────────┐
        │  ACQUIRE FIRST INFORMATION AND       │
        │       SECOND INFORMATION             │
        └─────────────────┬───────────────────┘
                          │
                          ▼               S602
        ┌─────────────────────────────────────┐
        │      GENERATE ARRANGEMENT            │
        │       PATTERN CANDIDATE              │
        └─────────────────┬───────────────────┘
                          │
                          ▼               S603
        ┌─────────────────────────────────────┐
        │      GENERATE CRYSTAL STRUCTURE      │
        └─────────────────┬───────────────────┘
                          │
                          ▼               S604
        ┌─────────────────────────────────────┐
        │      PREDICT PHYSICAL PROPERTY       │
        └─────────────────┬───────────────────┘
                          │
                          ▼               S605
        ┌─────────────────────────────────────┐
        │    NARROW DOWN CRYSTAL STRUCTURE     │
        └─────────────────┬───────────────────┘
                          │
                          ▼               S606
        ┌─────────────────────────────────────┐
        │      OUTPUT FOURTH INFORMATION       │
        └─────────────────┬───────────────────┘
                          │
                          ▼
                    ┌──────────────┐
                    │     END      │
                    └──────────────┘
```

# FIG. 30

(a)

## SELECT ELEMENT

| 1 | | | | | | | | | | | | | | | | | 18 |
|---|---|---|---|---|---|---|---|---|---|---|---|---|---|---|---|---|---|
| H | 2 | | | | | | | | | | | 13 | 14 | 15 | 16 | 17 | He |
| Li | Be | | | | | | | | | | | B | C | N | O | F | Ne |
| Na | Mg | 3 | 4 | 5 | 6 | 7 | 8 | 9 | 10 | 11 | 12 | Al | Si | P | S | Cl | Ar |
| K | Ca | Sc | Ti | V | Cr | Mn | Fe | Co | Ni | Cu | Zn | Ga | Ge | As | Se | Br | Kr |
| Rb | Sr | Y | Zr | Nb | Mo | Tc | Ru | Rh | Pd | Ag | Cd | In | Sn | Sb | Te | I | Xe |
| Cs | Ba | — | Hf | Ta | W | Re | Os | Ir | Pt | Au | Hg | Tl | Pb | Bi | Po | At | Rn |
| Fr | Ra | — | Rf | Db | Sg | Bh | Hs | Mt | Ds | Rg | Cn | Nh | Fl | Mc | Lv | Ts | Og |

| La | Ce | Pr | Nd | Pm | Sm | Eu | Gd | Tb | Dy | Ho | Er | Tm | Yb | Lu |
|---|---|---|---|---|---|---|---|---|---|---|---|---|---|---|
| Ac | Th | Pa | U | Np | Pu | Am | Cm | Bk | Cf | Es | Fm | Md | No | Lr |

HATCHED: ARRANGED AT CENTER OF POLYHEDRON
DOTTED: ARRANGED AT VERTEX OF POLYHEDRON

NEXT

(b)

## SELECT ARRANGEMENT OF ELEMENTS BELOW

| | VERTEX | POLYHEDRON CENTER | NUMBER |
|---|---|---|---|
| Cu | | ✔ | 1 |
| In | | ✔ | 1 |
| Se | ✔ | | 2 |

NEXT

(c)

## SELECT ONE THREE-DIMENSIONAL STRUCTURE

LOAD THREE-DIMENSIONAL STRUCTURE

49

# FIG. 31

GENERATION RESULTS (* RESULTS)

id:1
COMPOSITION : CuInSe2
SPACE GROUP : P -4m2
NUMBER OF ATOMS : 8

id:2
COMPOSITION : CuInSe2
SPACE GROUP : P4/mmm
NUMBER OF ATOMS : 8

id:3
COMPOSITION : CuInSe2
SPACE GROUP : I-42d
NUMBER OF ATOMS : 16

< 1 2 3 4 ..100 >

EXPORT SELECTED CRYSTAL STRUCTURE

FIG. 32

(a)

(b)

## INTERNATIONAL SEARCH REPORT

International application No.

**PCT/JP2023/001945**

### A. CLASSIFICATION OF SUBJECT MATTER

***G16C 20/30***(2019.01)i

FI: G16C20/30

According to International Patent Classification (IPC) or to both national classification and IPC

### B. FIELDS SEARCHED

Minimum documentation searched (classification system followed by classification symbols)

G16C10/00 - 99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | CrystalMaker: Overview, [online]. 22 January 2022., pp. 1-15, [retrieval date : 22 February 2023], <URL:https://web.archive.org/web/20220122203156/http://crystalmaker.com/crystalmaker/index.html><br>entire text, all drawings | 1-17 |
| A | Building a New Crystal. YouTube [online][video]. 31 July 2018., in particular, play time 0:00-3:54, [retrieval date: 03 March 2023], <URL:https://www.youtube.com/watch?v=AnYD0ERAfJw><br>in particular, play time 0:00-3:54 | 1-17 |
| A | JP 2020-106483 A (NIPPON STEEL CORP) 09 July 2020 (2020-07-09)<br>entire text, all drawings | 1-17 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **03 March 2023** | **14 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

International application No.

**PCT/JP2023/001945**

| Patent document cited in search report | Publication date (day/month/year) | Patent family member(s) | Publication date (day/month/year) |
|---|---|---|---|
| JP 2020-106483 A | 09 July 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20190286791

- JP 2021081769 A

**Non-patent literature cited in the description**

- **S. FREDERICKS** ; **K. PARRISH** ; **D. SAYRE et al.** *Computer Physics Communications*, 2021, vol. 261, 107810
- **KOTANI-SUNADA**. *Trans. Amer. Mat*, 2000

- **P.V. BUSHLANOV** ; **V.A. BLATOV** ; **A.R. OGANOV**. Topology-based crystal structure generator. *Computer Physics Communications*, 2019, vol. 236, 1-7
- **S. WENHAO et al.** The thermodynamic scale of inorganic crystalline metastability.. *Science advances*, 2016, vol. 2 (11), e1600225